# EUROPEAN PATENT SPECIFICATION

(11) **EP 1 756 160 B1**
(45) Date of publication and mention of the grant of the patent: **14.08.2013**
(21) Application number: 05752836.6
(22) Date of filing: 17.06.2005
(51) Int. Cl.: C07K 14/81, A61K 38/16, A61K 38/57, A61K 38/55

(54) **OLIGOMERIC PEPTIDES AND THEIR USE FOR THE TREATMENT OF HIV INFECTIONS**
OLIGOMERE PEPTIDE UND DEREN VERWENDUNG ZUR BEHANDLUNG VON HIV-INFEKTIONEN
PEPTIDES OLIGOMERES ET LEUR UTILISATION POUR TRAITER DES INFECTIONS A VIH

(30) Priority: 18.06.2004 EP 04014304; 18.06.2004 US 580384 P; 29.04.2005 US 675861 P
(43) Date of publication of application: 28.02.2007
(73) Proprietor: IPF Pharmaceuticals GmbH, 30625 Hannover (DE)
(72) Inventor: ADERMANN, Knut, 30177 Hannover (DE); KIRCHHOFF, Frank, 89077 Ulm (DE); MÜNCH, Jan, 89077 Ulm (DE); SCHULZ, Axel, 30161 Hannover (DE); FORSSMANN, Wolf-Georg, 30559 Hannover (DE)
(74) Representative: von Kreisler Selting Werner
(86) International application number: PCT/EP2005/052833
(87) International publication number: WO 2005/123771

(56) References cited:
- WO-A-01/34640
- WO-A-2004/056871
- PARK SANG-HYUN ET AL: "Genetic selection for dissociative inhibitors of designated protein-protein interactions" NATURE BIOTECHNOLOGY, vol. 18, no. 8, August 2000 (2000-08), pages 847-851, XP002318223 ISSN: 1087-0156

## Description

The present invention relates to oligomeric peptides which exhibit inhibitory activity on the infection of human cells by human immunodeficiency virus (HIV). The present invention also concerns antibodies which specifically bind to the oligomeric peptides, as well as a medicament comprising said peptides or antibodies. The use of the oligomeric peptides according to the invention for the manufacturing of a medicament for the treatment of HIV infections is also disclosed. The present invention also concerns an assay comprising at least one oligomeric peptide according to the invention, for determining molecules capable of interaction with the fusion peptide of HIV. Furthermore, the present invention discloses a diagnostic agent containing at least one of the oligomeric peptides according to the invention, or at least one antibody according to the invention. Also disclosed is the use of the diagnostic agent for testing isolated plasma, tissue, urine, semen and/or cerebrospinal fluid for HIV infection.

### Technical Background

In the last years, intensive research for therapeutics with activity against infection by HIV, was performed. Several medicaments were developed and tested, which delay and suppress the outbreak of AIDS and lower the level of the HIV in blood. In the US, the life-span of HIV-infected patients after the outbreak of AIDS was raised from 11 months in 1984 to 46 months in 1997.
In the search for therapeutics, various strategies were applied, which lead to several classes of medicaments such as the protease-blockers inhibiting a protease, which the virus requires for replication, and medicaments inhibiting the viral reverse transcriptase, which is essential for the replication of retroviruses. A group of active agents developed only recently are fusion inhibitors, which shall prevent the fusion of the virus with the host cells. It was also shown that the provision of interleukin-2 in combination with other active agents could increase the strength of the immune response.
Entry inhibitors block the attachment of HIV viral particles to blood cells by blocking one of the molecular steps occurring during viral attachment to and entry into the host cell. An important step is binding of HIV to one of the major chemokine coreceptors CCR5 and CXCR4 (CC chemokine receptor 5 and CXCR chemokine receptor 4). These coreceptors are located on the surface of blood cells and are required to bind to HIV envelope proteins before viral entry. Another step of viral interaction with cells required for entry is the binding of the HIV envelope protein gp120 to cellular CD4 receptors. These steps are often referred to as attachment of the viral particle to cellular targets. The blocking of the binding of HIV to chemokine coreceptors has been shown to suppress viral entry (Strizki J.M., Proc. Natl. Acad Sci. USA, 2001, . 98, 12718-12723). The same was reported by blocking the interaction of gp120 with CD4 receptors (Lin et al., Proc. Natl. Acad Sci. USA, 2003, 100, 11013-11018). The HIV protein gp41 has also been recognised as a potential target for anti-HIV drug development (Gordon et al., AIDS Research and Human Retroviruses 11, 677-686, 1995). The first approved fusion inhibitor is enfuvirtide (T-20, Fuzeon, DP178) (WO 01/51673 A2; WO 96/40191; Cervia J.S et al., Clin. Infect. Dis, 2003, 37, 1102-1106; Kilby J.M., Nature Medicine, 1998, 4, 1302-1307). This fusion inhibitor is identical to a part of the HIV envelope protein gp41 called HR-2, and inhibits HIV-cell fusion by binding to the HR-1 segment (HR = heptad repeat) of gp41, thus preventing the binding of HR-2 to the HR-1 segment of gp41 which in turn prevents the formation of a six-helix bundle required for fusion of the viral particle and the blood cell. T-20 has not been shown to bind to protein segments other than HR-1 of HIV gp41 or even other molecules of viral or eukaryotic origin. A further agent with biological activity against HIV was recently described in WO 01/34640. Disclosed is a peptide of 20 amino acids named VIRIP (virus inhibiting peptide), which was isolated from human hemofiltrate and found to inhibit the infection of human cells by HIV. Synthetic derivatives of VIRIP with biological activity against HIV are disclosed in WO 2004/056871.

WO 01/34640 discloses the peptide VIRIP with biological activity against HIV infection.

Park Sang-Hyun et al., Nature Biotec, 18, 847, disclose a peptidic inhibitor of HIV protease and disclose that the potency of said inhibitor can be 40-fold increased by forming a homodimer, using a linker that joins two peptides at the cysteine side chains.

Furthermore, WO 2004/056871 discloses peptides with biological activity against HIV infection.

Despite those efforts and different available medication, the problem remains unsolved that there is still no cure against AIDS, because the known therapeutics, though capable of significantly lowering the level of HIV in the body and of HIV-infected blood cells, do not remove the virus entirely. A special drawback is, that the HIV is especially prone to mutations, which often result in the development of resistance against certain therapeutics. In general, the known therapeutics are only sufficiently effective if they are administered in combination with other therapeutics. Such combined therapies at present extend the lifespan of the average patient without providing a cure, and are generally accompanied by severe side effects and frequently do not allow the patient to lead a "normal" life.
There is a great medical need to provide new therapeutics and improved therapeutics, which will lead to improved therapies, less side effects, and significant extension of the life expectancy of those infected by HIV, before or after the outbreak of AIDS.
The present invention faces the problem to provide new therapeutics, which will overcome the problems as described above, and will allow an efficient therapy or will contribute to an efficient combination therapy.

### Summary of the Invention

Surprisingly, the problem is solved by oligomeric peptides provided by the present invention, which interact at least with the fusion peptide of HIV gp41. The fusion peptide is the very amino-terminal part of gp41 consisting of about 30 amino acid residues. In a current model, the hydrophobic fusion peptide of gp41 serves as an anchor connecting the viral particle with the cellular host membrane (Dimitrov A.S. et al., Biochemistry, 2003, 42, 14150-14158; Mobley et al., Biochim. Biophys. Acta, 1999, 1418, 1-18), and the peptides of the present invention interfere with the HIV cell entry process, and thus prevent viral entry.

The present Invention discloses peptides with a biological activity against HIV infection, comprising monomeric peptide chains of amino acid sequence
(Z₁-LE-X₁-IP-X₂-X₃-X₄-P-X₅-X₆-X₇-X₈-X₉-X₁₀-K-X₁₁-X₁₂-X₁₃-X₁₄-X₁₅-Z₂)ₙ
wherein
n indicates the number of monomeric peptide chains, whereby n is 2, 3 or 4;
X₁ is a lysine, alanine, or aspartic add;
X₂ is a cysteine, methionine or isoleucine;
X₃ is a serine, cysteine, lysine or glycine;
X₄ is an isoleucine, alanine, phenylalanine or cysteine;
X₅ is a proline, D-proline or a substituted L-or D-proline;
X₆ is a cysteine or glutamic acid,;
X₇ is an amino acid with a hydrophobic or an aromatic side chain or cysteine;
X₈ is an amino acid with a hydrophobic or an aromatic side chain or cysteine;
X₉ is an amino acid with an aromatic side chain;
X₁₀ is a glycine, alanine or asparagine;
X₁₁ is a proline, aspartic acid, octahydroindolyl-2-carboxylic acid or D-1,2,3,4-tetrahydroisoquinoline-3-carboxylic acid;
X₁₂ is a phenylalanine, alanine, glycine, glutamic acid or D-1,2,3,4-tetrahydroisoquinoline-3-carboxylic acid;
X₁₃ is an amino acid with a hydrophobic or an aromatic side chain;
X₁₄ is an amino acid with a hydrophobic or an aromatic side chain;
X₁₅ is a phenylalanine or deletion;
Z₁ is NH₂ or a sequence of 1 to 10 amino acid residues;
Z₂ is COOH or a sequence of 1 to 10 amino acid residues;
and oligomeric peptides which are fragments and/or derivatives, especially amidated, alkylated, acylated, sulfated, pegylated, phosphorylated and/or glycosylated derivatives, and mutants thereof,
with the proviso that
(a) if X₁₂ is alanine, glycine, glutamic acid, or D-1,2,3,4-tertrahydroisoquinoline-3-carboxylic acid then X₁₃, X₁₄ and X₁₅ are phenylalanine, valine and phenylalanine respectively; and/or
(b) if X₁₂ is phenylalanine, then X₁₃, X₁₄ and X₁₅ are valine, phenylalanine and a deletion, respectively; and
(c) there are at maximum three cysteine residues in a peptide; and
(d) the oligomeric peptide is not (LEAIPCSIPPEFLFGKPFVF)₂ (VIR-576); and
(e) the monomeric peptide chains are not linked by peptide bonds between the N- terminus of one peptide chain to the C-terminus of another peptide chain.

Further disclosed are peptides with the generic formula (Z₁-LE-X₁-IP-X₂-X₃-X₄-P-X₅-X₆-X₇-X₈-X₉-X₁₀-K-X₁₁-X₁₂-X₁₃-X₁₄-X₁₅-Z₂)ₙ, X₇ is phenylalanine, cysteine, valine, isoleucine, leucine, 3,3-diphenylalanine, 1- naphthylalanine, or p-fluorophenylalanine; X₈ is a phenylalanine, leucine, alanine, tryptophane, glycine, cysteine, D-1,2,3,4-tetrahydrisoquinoline-3-carboxylic acid or L-1,2,3,4-tetrahydroisoquinoline-3-carboxylic acid; X₉ is a phenylalanine or D-1,2,3,4-tetrahydrolsoquinoline-3-carboxylic acid; and Z₁ is preferably NH₂ or a sequence of 1 to 3 amino acid residues and Z₂ is preferably COOH or a sequence of 1 to 3 amino acid residues. The biological activity against HI1/ infection of the above peptides, as measured as IC₅₀, is equal of or below of 6500 nM.

The invention discloses peptides with a biological activity against infection by HIV, are those having the amino acid sequence
(Z₁-LE-X₁-IP-X₂-X₃-X₄-P-X₅-X₆-X₇-X₈-X₉-X₁₀-K-X₁₁-FVF-Z₂)ₙ,
wherein
n indicates the number of monomeric peptide chains, whereby n is 2, 3 or 4;
X₁ is a lysine, alanine or aspartic acid;
X₂ is a cysteine, methionine or isoleucine;
X₃ is a serine, cysteine or glycine;
X₄ is an isoleucine or cysteine;
Xs is a proline, D-proline or any substituted L- or D-proline;
X₆ is a cysteine or glutamic acid;
X₇ is a phenylalanine, cysteine, valine, isoleucine or 3,3-diphenylalanine;
X₈ is a phenylalanine, leucine, alanine, glycine, cysteine, D-1,2,3,4-tetrahydroisoquinoline-3-carboxylic acid or L-1,2,3,4-tetrahydroisoquinoline-3-carboxylic acid;
X₉ is an amino acid with an aromatic side chain;
X₁₀ is a glycine or asparagine;
X₁₁ is a proline or D-1,2,3,4-tetrahydmisoquinoline-3-carboxylic;
Z₁ is NH₂ or a sequence of 1 to 10 amino acid residues;
Z₂ is COOH or a sequence of 1 to 10 amino acid residues;
and oligomeric peptides which are fragments and/or derivatives, especially amidated, alkylated, acylated, sulfated, pegylated, phosphorylated and/or glycosylated derivatives, and mutants thereof.
In a preferred embodiment of the above Oligomeric peptide with the generic formula (Z₁-LE-X₁-IP-X₂-X₃-X₄-P-X₅-X₆-X₇-X₈-X₉-X₁₀-K-X₁₁-FVF-Z₂)ₙ, X₉ is a phenylalanine or D-1,2,3,4-tetrahydruisoquinoline-3-carboxylic acid, Z₁ is preferably NH₂ or a sequence of 1 to 3 amino acid residues and Z₂ is preferably COOH or a sequence of 1 to 3 amino acid residues. The biological activity against HIV infection of the above oligomeric peptide, as measured as IC₅₀, is equal of or below of 2000 nM.

Even further dislcosed are oligomeric peptides with a biological activity against infection by HIV, which are those having the amino acid sequence
(Z₁-LE-X₁-IP-X₂-X₃-IP-X₅-X₆-X₇-X₈-F-X₁₀-KPFVF-Z₂)ₙ,
wherein
n indicates the number of monomeric peptide chains, whereby n is 2, 3 or 4;
X₁ is a lysine, alanine or aspartic acid;
X₂ is a cysteine, methionine or isoleucine;
X₃ is a serine or glycine;
X₅ is a L-proline, D-proline or any substituted L- or D-proline
X₆ is a cysteine or glutamic acid;
X₇ is a phenylalainine or valine;
X₈ is a phenylalanine, leucine, alanine or L-1,2,3,4-tetrahydroisoquinoline-3-carboxylic acid;
X₁₀ is a glycine or asparagine;
Z₁ is NH₂ or a sequence of 1 to 10 amino acid residues;
Z₂ is COOH or a sequence of 1 to 10 amino acid residues;
and oligomeric peptides which are fragments and/or derivatives, especially amidated, alkylated, acylated, sulfated, pegylated, phosphorylated and/or glycosylated derivatives, and mutants thereof.
Also disclosed is an oligomeric peptide with the generic formula (Z₁-LE-X₁-IP-X₂-X₃-IP-X₅-X₆-X₇-X₈-F-X₁₀-KPFVF-Z₂)ₙ, Z₁ is preferably NH₂ or a sequence of 1 to 3 amino acid residues and Z₂ is preferably COOH or a sequence of 1 to 3 amino acid residues. The biological activity against HIV infection of the peptide, as measured as TC₅₀, is equal of or below of 800 nM.

The invention discloses oligomeric peptides with biological activity against infection by HIV, which are those having the amino acid sequence
(Z₁-LEAIP-X₂-SIP-X₅-X₆-V-X₆-FNKPFVF-Z₂)ₙ,
wherein
n indicates the number of monomeric peptide chains, whereby n is 2, 3 or 4;
X₂ and X₆ are cysteines, or X₂ is methionine and X₆ is glutamic acid
X₅ is a D-proline or L-proline;
X₈ is an amino acid with a hydrophobic or an aromatic side chain or lysine;
Z₁ is NH₂ or a sequence of 1 to 10 amino acid residues;
Z₂ is COOH or a sequence of 1 to 10 amino acid residues;
and oligomeric peptides which are fragments and/or derivatives, especially amidated, alkylated, acylated, sulfated, pegylated, phosphorylated and/or glycosylated derivatives, and mutants thereof,
with the proviso that at least one of the following is true:
X₂ and X₆ are cysteines; or
X₅ is D-proline; or
X₈ is not lysine.
The peptide is disclosed with the generic formula (Z₁-LEAIP-X₂-SIP-X₅-X₆-V-X₈-FNKPFVF-Z₂)ₙ, Z₁ is preferably NH₂ or a sequence of 1 to 3 amino acid residues and Z₂ is preferably COOH or a sequence of 1 to 3 amino acid residues.
In the dislcosed oligomeric peptides of the monomeric peptides chains are crosslinked. They may be crosslinked either by direct intermolecular bonds between two amino acid side chains of two monomeric peptides or between the amino acid side chain of one monomeric peptide to the N- or C-terminus of another monomeric peptide. In another embodiment of the invention, the bonds are indirect bonds via bifunctional linker molecules.
Also disclosed are oligomeric peptides wherein the cysteine residues in the monomeric peptide chains at positions 6 and 11, 6 and 12, 7 and 12, or 8 and 13 are connected by an intramolecular disulfide bond. Thus the oligomeric peptides with cysteine residues at these positions may occur with an intramolecular bridge between these residues of one monomeric peptide chain, or with disulfide bonds between the monomeric peptide chains, i.e. the oligomerization is achieved via the disulfide bonds. Thus also are disclosed oligomeric peptides, wherein each monomeric peptide chain comprises two cysteine residues, wherein the oligomerization is achieved via the two cysteine residues which are linked by disulfide bonds to the two cysteine residues of the second monomeric peptide chain, resulting in a homo-dimer comprising the two monomeric peptide chains linked via two disulfide bonds. Also disclosed are oligomeric peptides with three cysteines in each monomeric peptide chain, wherein two cysteine residues in each monomeric unit are involved in an Intramolecular disulfide bond, and the third cysteine in each monomeric peptide chain takes part in the oligomerization, by forming a disulfide bond to the cysteine residue of the monomeric chain not taking part in the intramolecular disulfide bond. Further are disclosed oligomeric peptides with a single cysteine residue in each monomeric peptide chain, wherein said cysteine residues are connected to each other by a disulfide bond, thus linking the monomeric peptide chains of the oligomeric peptide. A homo-dimer formed in this way is preferred. The oligomeric peptides with cysteine residues in each monomeric peptide chain, at least one cysteine residue in each monomeric peptide chain is cross-linked via a bifunctional small organic spacer group containing two thiol groups to a cysteine residue in the other monomeric peptide chain of the oligomeric peptide. Dimers are the preferred embodiment of the oligomeric peptides comprising at least one cysteine residue.
An even further embodiment is an oligomeric peptide comprising at least one of the above described features, wherein in at least one of the monomeric peptide chains of the oligomeric peptide the leucine residue at amino acid position 1 and the glutamic acid at amino acid position 2 are covalently linked by an N-alkylated amide bond or by an ester bond or by a reduced peptide bond or by a retro-inverso peptide bond or by an N-alkylated retro-inverso peptide bond. Preferred are dimers, wherein the leucine residue at amino acid position 1 and the glutamic acid at amino acid position 2 are covalently linked by an N-alkylated amide bond or by an ester bond or by a reduced peptide bond or by a retro-inverso peptide bond or by an N-alkylated retro-inverso peptide bond.
Also an embodiment is an oligomeric peptide with at least one of the above described features, comprising 2, 3 or 4 repetitive units of a monomeric peptide chain in a linear order, whereby the monomeric peptide chains are covalently linked via an indirect peptide bond between Z₂ and Z₁ of the consecutive monomeric peptide chains. An indirect peptide bond occurs, if a small organic bifunctional spacer with an amino group and a carboxyl group connects Z₂ with Z₁ of consecutive monomeric peptide chains. In such a linear oligomeric peptide with an indirect peptide bond, the repetitive units are either all identical monomeric peptide chains, resulting in a homogeneous oligomeric peptide, or the repetitive units are selected from differing monomeric peptide chains, such that a tetramer can comprise 1, 2, 3 or 4 non-identical monomeric peptide chains, a trimer 1, 2 or 3 non-identical monomeric peptide chains, or a dimer 2 non-identical monomeric peptides chains, resulting in a heterogeneous oligomeric peptide. Preferred linear oligomeric peptides with an indirect peptide bond are such with two repetitive units, i.e. dimers. Also an embodiment are cyclic oligomeric peptides, in particular those with 2, 3 or 4 monomeric peptide chains. One form of a cyclic oligomeric peptide comprises repetitive units of a monomeric peptide chain, whereby the first and all consecutive monomeric peptide chains are covalently linked via a direct or an indirect peptide bond between Z₂ and Z₁ of the consecutive monomeric peptide chains, and at least one further covalent bond between the first and the second and/or the first and the third and/or the first and the forth monomeric peptide chains, and/or the second with the third and'/or the second with the forth monomeric peptide chains, and/or the third with the forth monomeric peptide chains occurs. The at least one further covalent bond may be selected from the group of amide bonds, in particular peptide bonds such as they occur between side chains of amino acid residues with acidic and basic amino acids, or the amino-terminus of the first monomeric peptide chain and the carboxyl-terminus of the last monomeric peptide chain, oxime bonds, hydrazone bonds, thiazolidine bonds, thioester, bonds, ether bonds and disulfide bonds between cysteine residues, etc.
A further embodiment according to the disclosure is anyone of the above described linear oligomeric peptides comprising at least one cysteine residue through which either a direct disulfide bond is formed to a cysteine residue of another oligomeric peptide, including a linear oligomeric peptide, with at least one cysteine residue, or where said cysteine residues are linked to each other via a short organic bifunctional spacer with two thiol groups. Instead, or in addition to a disulfide bridge between the above described linear oligomeric peptides to another oligomeric peptide, the linkage between these peptides is established through at least one amide bond formed between one carboxyl group and one amino group of amino acid residues with acidic and basic side chains, respectively. Said acidic and basic side chains may alternatively be connected via a short organic bifunctional spacer comprising an amino group as well as a carboxyl group.
A further embodiment of oligomeric peptides according to the disclosure those, wherein the monomeric peptide chains are covalently linked to each other via at least one amide bond between two monomeric peptide chains, which is achieved by a covalent bond between a side chain amino group of a basic amino acid in one monomeric peptide chain to a side chain carboxyl group of an acidic amino acid In another monomeric peptide chain. In particular such amide bonds can form between the side chains of a lysine residue with the side chain of an aspartate residue, and/or between the side chains of a lysine residue and a glutamate residue. In a further embodiment, oligomerization of these peptide chains is achieved indirectly with a bifunctional small organic spacer group containing one amino group and one carboxyl group, which crosslinks these monomeric peptide chains by covalently binding to the carboxyl side chain of an acidic amino acid of the one peptide chain with its amino group and to a side chain amino group of a basic amino acid of the second peptide chain with its carboxyl group. Tetramers, trimers and dimers can be formed via a direct or indirect amide bond between side chains of acidic and basic amino acids of monomeric peptide chains, whereby dimers are the preferred embodiment. Also an embodiment are oligomeric peptides which contain up to four peptide chains covalently linked by a peptide bond via their carboxy-terminus to the amino functions of an amino acid containing two amino groups, such as lysine and the lower lysine derivates. A further embodiment is an oligomeric peptide, which comprises two or four monomeric peptide chains and at least one lysine-core, which covalently links the monomeric peptide chains. The lysine core functions as a linking molecule (which can also be described as a spacer) and has the formula "KX", wherein "X" is either an amino acid, a pegyl group or a deletion, i.e. "X" is missing. The lysine core links two monomeric peptide chains via formation of two peptide bonds with the carboxy-termini of each monomeric peptide chain. The preferred amino acid in the lysine core is an alanine, and the preferred pegyl group is a mini-PEG. A lysine core can be linked over an amino acid with amino groups in its side chains, such as a lysine residue or a lower lysine homologue, to another lysine core via a peptide bond. If both lysine cores covalently link two monomeric peptide chains, the resulting oligomeric peptide comprises 4 monomeric peptide chains. The lysine core is also referred to as dendrimeric structure, and the monomeric peptide chains are said to be linked to the dendrimeric lysine core structure. A further embodiment are oligomeric peptides according to the invention wherein the monomeric peptide chains are linked via at least one oxime, hydrazone and thiazolidine bond, using reactive side chains of natural and non-natural amino acids. Oxime and hydrazone-linkages can occur between an aldehyde functionality generated from an amino acid with a hydroxy group in the side chain such as serine and a hydroxylamine or hydrazine side chain, introduced through special amino acids such as amino oxyacetic acid, aminoserine or hydrazino carboxylic acid. The thiazolidine formation can occur between an N-terminal cysteine residue and aldehyde side chains (examples: Chan W.C. et al. (editors), Fmoc solid phase peptide synthesis: A practical approach, Oxford University Press, Oxford, 2000, p 243-263; Novabiochem 2004/5 Catalogue, Reagents for Peptide and High-Throughput Synthesis, p.5.2-5.3). Also an embodiment according to the invention is an oligomeric peptide, wherein each monomeric peptide chain comprises at least one amino acid residue with a sulphur atom in its side chain, enabling a covalent linkage between the monomeric peptide chains via a thioether bridge. Of those oligomeric peptides connected via at least one thioether bridge, those where two monomeric peptide chains are connected, i.e. dimers, are preferred. Those oligomeric peptides, wherein the monomeric peptide chains are linked to each other via a bifunctional spacer molecule selected from the group of organic spacers with two thiol groups, organic spacers with two amino groups, organic spacers with two carboxyl groups and organic spacers with one carboxyl groups and one amino group are a further preferred embodiment. In particular those oligomeric peptides with two monomeric peptide chains, i.e. dlmers.
The oligomeric peptides of the invention interact with the HIV fusion peptide of gp41, characterised by an IC₅₀ of equal or below 6500 nM, such as VIR-574 (SEQ ID NO. 2) or VIR-577 (SEQ ID NO. 3), preferably an IC₅₀ of equal or below 2000 nM and most preferably an IC₅₀ of equal or below 800 nM, such as VIR-673 (SEQ ID NO. 4) with an IC₅₀ of 607 nM. The nucleic acids encoding the monomeric peptide chains of VIR-674, VIR-675, VIR-676, VIR-677, VIR-678, VIR-679, VIR-680, VIR-681, VIR-682, VIR-683, VIR-684, VIR-685, VIR-686, VIR-687, VIR-688, VIR-689, VIR-690, VIR-691, VIR-692, VIR-693 and VIR-694 which correspond to VIR-166, VIR-175, VIR-261, VIR-273, VIR-274, VIR-344, VIR-345, VIR-348, VIR-352, VIR-353, VIR357, VIR-358, VIR-448, VIR-449, VIR-454, VIR-455, VIR-483, VIR-484, VIR-512, VIR-568 and VIR-580, respectively, in WO 2004/056871, are not part of the invention. Further embodiments are antibodies binding specifically to these oligomeric peptides of the invention. The antibodies are specific, because they recognize the oligomeric peptides, but not the isolated monomeric peptide chains. Therefore, the antibody binding site comprises a part of the molecule, which is different from one single monomeric chain. For instance, the binding site might comprise the linkage between two monomeric peptide chains.
A further embodiment is a medicament comprising anyone of these oligomeric peptides, or specific antibodies directed against these oligomeric peptides. In one embodiment the medicament is in galenic formulations for oral, intravenous, intramuscular, intranasal, intracutaneous, subcutaneous and/or intrathecal administration. A further embodiment is said medicament comprising at least one further therapeutic agent. Also an embodiment is the medicament, wherein the said at least one further therapeutic agent is a viral protease inhibitor, a reverse transcriptase inhibitor, a fusion inhibitor, a cytokine, a cytokine inhibitor, an integrase inhibitor, a glycosylation inhibitor or a viral mRNA inhibitor, etc. Use of these oligomeric peptides for the manufacturing of a medicament for the treatment of HIV infections is a further embodiment. Also an embodiment is an assay for determining molecules capable of interacting with the fusion peptide of HIV, comprising anyone of the above oligomeric peptides of the invention. Use of these oligomeric peptides in said assay is also an embodiment. A further embodiment is a diagnostic agent comprising anyone of the above described oligomeric peptides or antibodies. One more embodiment is use of the diagnostic agent for assay systems for testing isolated plasma, tissue, urine, semen and/or cerebrospinal fluid levels for HIV infection.

### Detailed Description of the Invention

The oligomeric peptides of the present invention are related to the hemofiltrate-derived peptide VIRIP (SEQ ID No. 1) as disclosed and described in WO01/34640, and the peptides disclosed and described in PCT/EP03/15654, which have biological activity in preventing infection by HIV. The peptides of the present invention differ from the peptides disclosed and described in WO01/34640 PCT/EP03/15654 in that they are all of oligomeric structure composed of at least two single monomeric peptide chains.
The monomeric peptide chains are linked by at least one covalent bond. The covalent bonds can be a disulfide bond, an amide bond, in particular a peptide bond, a thioether bond, an ether bond or an oxime, hydrazone or thiazolidine bond. To generate an oligomeric peptide of the invention, monomeric peptide chains are oligomerized by connecting at least two single monomeric peptide chains using reactive groups of the amino acid side chains or the terminal functional groups. The single peptide chains are either chemically synthesized or biotechnologically produced, i.e. by expressing the nucleic acids coding for these single peptide chains in microorganisms such a bacteria, e.g. *E. coli,* fungi, e.g. yeast, and/or mammalian cells in culture, and subsequently covalently linking them to oligomers. Oligornetic peptides can also be synthesized as dendrimeric structures, which contain amino acids or a group containing at least two amino groups to which the monomeric peptide chains are coupled using solid-phase peptide synthesis technologies. Examples of such groups are ornithine, lysine, diaminobutanoic acid and diaminopropionic acid (Novabiochem 2004/5 Catalogue, Reagents for Peptide and High-Throughput Synthesis, p.26-29).
Oligomeric peptides of the invention also comprise polypeptides that are linearly assembled from repeating units of monomeric peptide chains via indirect peptide bonds, wherein the repeating units corresponds to monomeric peptide chains. An indirect peptide bond occurs, if a small organic bifunctional spacer with an amino group and a carboxyl group connects Z₂ with Z₁ of consecutive monomeric peptide chains
The oligomeric peptide with the sequence (LEAIPCSIPPEFLFGKPFVF)₂ (VIR-576) disclosed in PCT/EP03/15654, wherein the dimerization is achieved via a disulfide bond between the cysteine residues at position 6 of the two monomeric peptide chains, is not an oligomeric peptide according to the invention. However, the dimer with the sequence (LEAIPCSIPPEFLFGKPFVF)₂ (SEQ ID: NO. 56), wherein the dimerization is achieved in any other way, in particular through any one of the glutamate residues and a small bifunctional linker with two amino groups, which links the two monomeric chains by formation of two amide bonds at the glutamate residues, is part of the present disclosure. Also disclosed is a dimer with the sequence (LEAIPCSIPPEFLFGKPFVF)₂ (SEQ ID: NO. 57), wherein the dimerization is achieved through the lysine residue at amino acid position 16 and a small bifunctional linker with two carboxyl groups, which links the two monomeric chains by formation of two amide bonds at the two lysine residues.
The monomeric peptide chains of the oligomeric peptides according to the invention all differ from wild-type VIRIP (SEQ ID NO.: 1) at least in amino acid position 13, wherein VIRIP contains a lysine residue, while the monomeric peptide chains of the oligomeric peptides of the present invention do not contain a lysine residue at amino acid position 13. In addition to that, the monomeric peptide chains of the oligomeric peptides of the present invention have further amino acid changes throughout their 20 or 21 amino acids in comparison to VIRIP (SEQ ID NO.:1). The oligomeric peptides of the present invention all posses significantly higher anti-HIV activity (measured as IC₅₀ against two HIV-1 strains) than VIRIP (SEQ ID NO. 1). The monomeric peptide chains of the oligomeric peptides of the invention are based on an amino acid sequence of 20 or 21 amino acids, with possible extensions of 1 to 10 amino acids at both ends according to Z₁ and Z₂, whereby an extension of 3 amino acids is preferred. The amino acid numbering used herein always corresponds to the amino acids 1 to 21 of the basic sequence irrespective of a possible N-terminal extension due to a residue Z₁, such that amino acid position 1 corresponds to leucine and amino acid position 21 to phenylalanine or a deletion. The common amino acid one and three letter codes are used. If not indicated otherwise, the L-enantiomers of amino acids were used. The small letter "p" stands for D-proline. Other D-enantiomers are indicated by a "D-" prefix. 'Tic" stands for tetrahydrosioquinoline carboxylic acid. "Oic" stands for octahydroindole carboxylic acid.
The term "oligomeric peptide" refers to a peptide comprising in particular two, three or four monomeric peptide chains, which are covalently linked to each other. A dimer, that is an oligomeric peptide formed by two covalently linked monomeric peptide chains, is preferred. Said dimer comprises at least one covalent bond between the monomeric peptide chains, but may also have more covalent bonds. An oligomeric peptide with three monomeric peptide chains is termed trimer. An oligomeric peptide with four monomeric peptide chains is termed tetramer. In trimers the minimum number of linking covalent bonds is at least two, but may be higher, and tetramers do comprise at least three covalent bonds.
The term "monomeric peptide chain" refers to a peptide unit of the oligomeric peptide, and is an amino acid sequence of 20 or 21 amino acids, with possible extensions of 1 to 10 amino acids at both ends according to Z₁ and Z₂, whereby an extension of 3 amino acids is preferred. An oligomeric peptide of the invention comprises n monomeric peptide chains.

The term "oligomeric peptide" as symbolized by the formula (sequence)ₙ refers to a molecule formed by n peptides covalently linked to each other. As used herein, it is clear that "oligomeric peptide" is not restricted by a specific type of linkage between the monomeric peptide chains. The linkage might for example be a cysteine bridge or a linkage involving a lysine side chain. The term oligomeric peptide" and the formula (sequence)" in the context of the invention are thus not to be understood in the sense that the n amino acid monomers are all linked by conventional peptide bonds between the N- and the C-terminus.
The term "crosslinked" refers to any intermolecular covalent bond between two monomeric peptides which is different from the conventional peptide bond between the N-terminus of one monomeric peptide and the C-terminus of another monomeric peptide.
The term "covalently linked" is readily understood by the skilled person and refers to bonds like amide, thiol- thioester, ether- oxime, hydrazone and thiazolidine bonds, which are introduced in order to generate an oligomeric peptide. In particular it refers two, three or four monomeric peptide chains covalently linked to each other, thus giving rise to dimers, trimers and tetramers, respectively. The oligomeric peptides of the invention are described as "covalently linked", however this does not require each monomeric peptide chain of the oligomeric peptide to be linked to every other monomeric peptide chain in the oligomeric peptide; dimers are covalently linked by at least one of the above listed chemical bonds; trimers are covalently linked by at least two of the above listed chemical bonds, thus it is sufficient for the first monomeric peptide chain to be linked to the second monomeric peptide chain, and the second to be linked to the third monomeric peptide chain; tetramers are covalently linked by at least three of the above listed chemical bonds, thus it is sufficient for the first monomeric peptide chain to be linked to the second monomeric peptide chain, and the second to be linked to the third monomeric peptide chain, and the third monomeric peptide chain to be linked to the forth monomeric peptide chain. The monomeric peptide chains in the oligomeric peptide can have an identical or a different amino acid sequence. Oligomeric peptides made up of identical monomeric peptide chains are also referred to as "homogeneous" oligomeric peptides, and in case of dimers they are also referred to as "homo-dimers". If the monomeric peptide chains in the oligomeric peptide are different, the term "heterologous" is applied to the oligomeric peptide and its monomeric peptide chains. The covalent bond can be a "direct bond" between the respective peptide chains such as a disulfide bond, thioether bond, ether bond, amide bond, oxime bond, thiazolidine bond, hydrazone bond, i.e. there is no bridging spacer molecule. The peptide chains can also be covalently linked by a spacer of any chemical nature (Houben-Weyl, Methods of organic chemistry, Synthesis of peptides and peptidomimetics, Georg Thieme Verlag, Stuttgart 2002), the monomeric peptide chains are then said to be linked via an "indirect bond". Preferred are small organic bifunctional spacers such as those with two thiol groups, with two amino groups, with two carboxyl groups and those with one carboxyl group and one amino group.
The term "hydrophobic amino acid" as used herein is readily understood by the skilled person. In particular, it refers to any of the naturally occurring amino acids glycine, alanine, valine, leucine, isoleucine, methionine, proline, phenylalanine, tyrosine, tryptophane, and non-endogenous hydrophobic amino acids. Similar synthetic non-endogenous amino acids mimicking the behaviour of naturally occurring amino acids can be used as well, so long as the biological activity against HIV is not significantly lowered.
The term "aromatic amino acid" as used herein is readily understood by the skilled person. In particular it refers to any of the amino acids phenylalanine, tyrosine, tryptophane, histidine, and non-endogenous aromatic amino acids, such as 1-naphthylaianine, 3,3-diphenylalanine, p-fluorophenylalanine, or D-1,2,3,4-tetrahydroisoquinoline-3-carboxylic acid or L-1,2,3,4-tetrahydroisoquinoline-3-carboxylic acid, etc. Similar synthetic non-endogenous amino acids mimicking the behaviour of such amino acids can be used as well, so long as the biological activity against HIV is not significantly lowered.
The term "acidic amino acid side chain" is readily understood by the skilled person. In particular, it refers to the carboxylic acid side chain of glutamic acid and aspartic acid and any other natural and unnatural amino acid carrying a carboxylic acid as a side chain functionality, such as 2-amino adipinic acid. The term "basic amino acid side chain" is readily understood by the skilled person. In particular, it refers to the side chain amino group of lysine, ornithine or any other natural and unnatural amino acid carrying a amino functionality as a side chain, such as diaminopropionic acid. Also amino acids with basic side chains to which the present invention refers are the lower lysine derivatives, which possess a shortened side chain of only three, two or only one carbon atom (in comparison to lysine), which carries the amino group.
The term "unnatural amino acid" is readily understood by the skilled person. In particular it refers to any amino acid that does not belong to the 20 endogenous occurring amino acids which are: glycine, cysteine, alanine, valine, lysine, leucine, isoleucine, glutamic acid, aspartic acid, glutamine, asparagine, arginine, histidine, tyrosine, phenylalanine, tryptophane, serine, threonine, proline and methionine.

The term "cyclic" is readily understood by the skilled person. In particular it refers to oligomeric peptides that contain a cyclic structural motif in at least one of its monomeric peptide chains or a cyclic structural motif including at least two monomeric peptide chains. The cyclization can be achieved by backbone cyclization or by linking a side chain of an amino acid to a side chain of a different amino acid present in the same molecule. In a preferred embodiment of the invention, two cysteine residues, or one carboxylic acid side chain and one amino group-containing side chain form a cyclic motif via a disulfide bond or an amide bond, respectively.
The expression "biological activity against infection by HIV" refers to the ability of the oligomeric peptides to at least interfere with or prevent virus entry into mammalian cells. More specifically, it refers to its ability to interact with the fusion peptide of HIV gp41. A measure for the biological activity against infection by HIV is the IC₅₀ value.Preferrably, the biological activity of an oligomeric peptide of the invention against HIV infection is such that its IC₅₀ value is lower than the IC₅₀ value of the wild-type VIRIP.

Oligomeric peptides of the invention show favourable properties regarding the antiviral activity against infection with HIV and the stability in blood plasma when compared to the original VIRIP (SEQ ID NO.: 1) or monomeric analogues. Dimeric peptides have superior qualities. To obtain dimeric peptides of the invention, monomeric peptides chains are chemically linked introducing a covalent bond between the two peptide chains. The covalent link can be a direct bond between side chain functional groups such as the thiol group of cysteine residues, or a bond involving a spacer between the peptide chains as is present when two identical chains of a peptide are bound to the two amino groups of a lysine residue. The latter is often referred to as the smallest form of a lysine-core dendrimer (Sadler K., J. Biotechnology, 2002, 90, 195-229). The two peptide chains can also be connected via an amide bond between two single peptide chains, which is achieved by a covalent bond between a side chain amino group of one peptide chain to a side chain carboxyl group of a second chain. This linkage can also be achieved by using a small bifunctional organic spacer molecule which crosslinks two peptide chains by a covalent binding to the carboxyl side chain of one peptide chain and to a side chain amino group of a second peptide chain. Furthermore this organic spacer molecules can be used to crosslink two identical peptide chains by an amino functionality, if the spacer molecule contains two acid functionalities or to link two identical peptide chains by an carboxyl functionality, if the spacer molecule contains two amino functionalities (Pennington et al. (editors), Peptide synthesis protocols, Humana Press, Totowa 1994). The two peptide chains of the monomers can also be ligated via oxime, hydrazone and thiazolidine formation using reactive side chains of natural or non-natural amino acids (Rose K., J. Am. Chem. Soc., 1994, 116, 30-33; Shao, J. & Tam J.P., 1995, J. Am. Chem. Soc., 117, 3893-3899; Fisch I. et al, Bioconjugate Chem., 1992, 3, 147-153; Liu C.-F. & Tam J.P., Proc. Natl. Acad. Sci. USA, 1994, 91, 6584-6588). In addition, oligomeric peptides of the invention are obtained by solid-phase peptide synthesis of a linear oligomeric peptide containing repeating units of monomeric peptide chains.

The dimeric peptides of the invention, are structurally and biologically more stabile, and more active in comparison to the wild-type VIRIP (SEQ ID NO-1). They show an increased half-life leading to a higher concentration in plasma. In addition, they cause an increase of the local concentration of the antivirally active peptide chain at the site of action. The oligomeric peptides of the invention thus are molecules which interact more favourable with a viral receptor molecule, causing a more effective blocking of viral entry.

It also was found that by specifically varying the amino add sequence of the monomeric peptide chains in comparison to the wild-type VIRIP (SEQ ID NO. 1), oligomeric peptides with a significantly increased activity against HIV were obtained. The most significant increase in activity is observed in oligomeric peptides, when in the monomeric peptide chain the L-proline at position 10 is substituted by a D-proline, and/or two cysteines are introduced at amino acid positions 6 and 11, and/or when the positively charged lysine at position 13 is exchanged against an amino acid with a hydrophobic or aromatic side chain. It is believed that the increase in activity in comparison to the wild-type VIRIP (SEQ ID NO.1) is due to a change in structure. Cysteine bridges are known to influence the structure and to reduce the flexibility of a peptide significantly, as well as the introduction of a D-proline, which causes a change in secondary structural elements of a peptide and thus a changed orientation of different parts of the peptide to each other. Furthermore, the exchange of a lysine against an uncharged hydrophobic or aromatic amino acid will alter the structure, because a possible interaction of the positively charged lysine side chain with the negatively charged amino acids at positions 2 and 11 of the same molecule, or with a negatively charged portion of a receptor molecule is changed. A significant increase in the anti-HIV activity is further observed when the alanine residue at position 3 of a monomeric peptide chain of the oligomeric peptide is exchanged to a positively or negatively charged residue by substitution with lysine or aspartic acid residues. The introduction of a charged residue at position 3 can enhance the binding strength to a corresponding part of a receptor molecule by increased electrostatic or dipolar forces. The exchange of the amino acid residues at positions 7 and/or 15 of the monomeric peptide chains of the oligomeric peptide against a small amino acid residue, in particular glycine, has also been found to increase the anti-HIV activity of the oligomeric peptides according to the invention. Glycine residues are the least sterically hindering residues and allow an optimal internal structural arrangement of a peptide when binding to a receptor molecule or when forming aggregates with themselves required for binding with a receptor molecule. The described substitutions may be combined in monomeric peptide chains of the oligomeric peptides of the invention.

Monomeric chains of peptides of the invention can be chemically synthesized or produced by recombinant expression of the nucleic acids coding for the monomeric peptide chains according to the invention. Due to the small size, i.e. the low number of amino acid in the monomeric peptide chains contained in the oligomeric peptides of the invention, the entire peptide synthesis technologies can be utilised to chemically synthesise such monomeric peptide chains. In comparison to the synthesis of the HIV fusion inhibitor T-20, which requires the synthesis of three individual fragments, and subsequently the joining of the three fragments to give rise to the final product T-20, the monomeric peptide chains of the present invention, can be synthesized at large scale by stepwise solid phase methods or by solution phase chemistry. Thus the process to obtain monomeric peptide chains of the oligomeric peptides of the present invention is straightforward and therefore the costs of goods for the oligomeric peptides of the present invention are low. The covalent linking of the monomeric peptides is also straightforward, i.e. does not require difficult chemical reactions, and therefore the linking reaction does not greatly contribute to the costs of the final products comprising the oligomeric peptides.
A further advantage of the oligomeric peptides of the present invention is their solubility and stability over a broad range of pH (pH 2 - 8.5) in solvents of different ionic strength. In particular their stability in blood plasma has to be stressed. The chemical synthesis can be carried out on a solid support using solid-phase technologies or in solution phase, both being standard methods known to the skilled person. The monomeric peptide chains of the oligomeric peptides according to the invention can also be synthesized by the ligation of two or more side chain-protected or side chain-unprotected fragments, standard methods known to the skilled person (Tam J.P., Biopolymers, 2001, 60, 194-205). The solid-phase synthesis of monomeric peptide chains of the oligomeric peptides according to the invention or its fragments can be carried out using the Fmoc/tBu- or Boc/Bzl-protection pattern of amino acids. Other protective groups that are not in the standard Fmoc-protection scheme can be used. Purification of synthetic monomeric peptide chains and oligomeric peptides is achieved by chromatographic methods such as reverse-phase, ion exchange or size-exclusion. The methods for the chemical synthesis of the monomeric peptide chains and the oligomeric peptides of the invention mentioned here, are surveyed in several review publications (examples: Chan W.C. et al. (editors), Fmoc solid phase peptide synthesis: A practical approach, Oxford University Press, Oxford, 2000; Seewald N. et al., Peptides: biology" and chemistry, Wiley-VCH, Weinheim, 2002; Goodman M., Houben-Weyl, Methods of organic chemistry, Synthesis of peptides and peptidomimetics, Georg Thieme Verlag, Stuttgart 2002).
The introduction of a disulfide bond into a monomeric peptide chain of the oligomeric peptides of the invention may be achieved by applying oxidative chemical methods to monomeric peptide chains containing at least two cysteine residues, whereby the methods are known to the skilled person (Pennington et al. (editors), Peptide synthesis protocols, Humana Press, Totowa 1994; Chan W.C. et al. (editors), Fmoc solid phase peptide synthesis: A practical approach, Oxford University Press, Oxford, 2000). Disulfide bonds between monomeric peptide chains of the oligomeric peptides of the invention may be generated from reduced precursor monomeric peptide chains containing at least one unprotected cysteine residue, obtained from solid-phase or solution synthesis, by oxidative treatment. As oxidizing agents oxygen, dimethylsulfoxide, iron(III) salts, iodine, or others may be used. Disulfides of the monomeric peptide chains of the oligomeric of the invention may alternatively be introduced into the peptides from precursors containing protective groups at the corresponding cysteine residues. As protective groups acetamidomethyl, tert-butyl, S-tert-butyl or others may be used. Cleavage of protective groups and intra-chain disulfide bond formation may be carried out using agents such as iodine, phosphines, or others.
Cyclic monomeric peptide chains of the oligomeric peptides other than those with a disulfide bond can be obtained via backbone cyclization of the monomeric peptide chain or via a chemical bond between at least one reactive side chain group such as amino, carboxy, hydroxy or thio and any other reactive group present in the same molecule, as known to the skilled person (U et al., Curr. Top. Med. Chem., 2002, 2, 325-341; Tam J.P., Biopolymers, 2001, 60, 194-205; Goodman M., Houben-Weyl, Methods of organic chemistry, Synthesis of peptides and peptidomimetics, Georg Thieme Verlag, Stuttgart 2002).
Covalently linked oligomers of the monomeric peptides are obtained by linking two peptide chains via different types of chemical bonds. Disulfide-linked oligomers are synthesized by coupling two peptide chains either via activated cysteines or without any preactivation of the cysteines (Sacca B. et al., J. Pept. Sci., 2002, 8, 192-204; Seewald N. et al., Peptides: biology and chemistry, Wiley-VCH, Weinheim, 2002). Thioether bonds and ether bonds and peptide bonds between two peptide chains can be introduced according to different methods known to the skilled person and described in the literature (Seewald N. et al., Peptides: biology and chemistry, Wiley-VCH, Weinheim, 2002). Lysine-core dendrimers can be synthesized by coupling Fmoc-Lys(Fmoc)-OH to a solid support. After deprotection of the amino acid solid phase peptide synthesis leads to the oligomeric peptides (Seewald N. et al., Peptides: biology and chemistry, Wiley-VCH, Weinheim, 2002; Chan W.C. (editors) Fmoc solid phase peptide synthesis: A practical approach, Oxford University Press, Oxford 2000). Between the carboxy-terminal lysine residue and the peptide chains additional lysine residues can be placed. Lysine can be replaced by any other amino acid containing two amino groups such as ornithine and any unnatural amino acid carrying an amino group in the side chain, such as diaminopropionic acid.
In a preferred oligomeric peptide according to the invention, the connection of the two peptide chains via an amide bond between two single peptide chains is achieved by a covalent bond between a side chain amino group of one peptide chain to a side chain carboxyl group of a second chain. This linkage can also be achieved by using a small bifunctional organic spacer molecule which crosslinks two peptide chains by a covalent binding to the carboxyl side chain of one peptide chain and to a side chain amino group of a second peptide chain. Furthermore this organic spacer molecule can be used to crosslink two identical peptide chains by an amino functionality, if the spacer molecule bears two acid functionalities or to link two identical peptide chains by an carboxyl functionality, if the spacer molecule contains two amino functionalities. This methods are known to the skilled person and described in the literature (Pennington et al. (editors), Peptide synthesis protocols, Humana Press, Totowa 1994).
The ligation of two monomeric peptide chains inducing an oxime, a hydrazone or a thiazolidine formation using reactive side chains of natural or non-natural amino acids can be achieved by ligating a monomeric peptide with a hydroxylamine or hydrazine group to a peptide carrying an aldehyde group, or by coupling a peptide with an N-terminal cysteine to a peptide aldehyde, methods known to the skilled person and described in the literature (Chan W.C. et al. (editors), Fmoc solid phase peptide synthesis: A practical approach, Oxford University Press, Oxford, 2000, p 243-263; Novabiochem 2004/5 Catalog, Reagents for Peptide and High-Throughput Synthesis, p.5.2-5.3; Rose K., J. Am. Chem. Soc., 1994, 116, 30-33; Shao, J. & Tam J.P., 1995, J. Am. Chem. Soc., 117, 3893-3899; Fisch L, et al., Bioconjugate Chem., 1992, 3, 147-153; Liu C.-F. & Tam J.P., Proc. Natl. Acad. Sci. USA, 1994, 91, 6584-6588).
In the following paragraph it is briefly described, how the various oligomeric peptides of the invention are covalently linked. These peptides all present dimers.
VIR-674: linked via two cysteine bridges between Cys6 and Cysl1 of one monomeric peptide chain to Cys6 and Cys11, respectively, of a second identical monomeric peptide chain.
VIR-675: linked via an amide bond between Glull of the one monomeric peptide chain and Lysl6 of the second identical monomeric peptide chain. VIR-676: linked via an amide bond between Glull of one monomeric peptide chain and Lysl6 of the second identical monomeric peptide chain.
VIR-677: linked via two cysteine bridges between Cys7 and Cysl2 of one monomeric peptide chain to Cys7 and Cysl2, respectively, of a second identical monomeric peptide chain.
VIR-678: linked via two cysteine bridges between Cys7 and Cysl2 of one monomeric peptide chain to Cys7 and Cysl2, respectively, of a second identical monomeric peptide chain.
VIR-679: linked via two cysteine bridges between Cys6 and Cysll of one monomeric peptide chain to Cys6 and Cys11, respectively, of a second identical monomeric peptide chain.
VIR-680: linked via two cysteine bridges between Cys6 and Cysl1 of one monomeric peptide chain to Cys6 and Cys11, respectively, of a second identical monomeric peptide chain.
VIR-681: linked via two cysteine bridges between Cys6 and Cys11 of one monomeric peptide chain to Cys6 and Cys11, respectively, of a second identical monomeric peptide chain.
VIR-682: linked via two cysteine bridges between Cys6 and Cys11 of one monomeric peptide chain to Cys6 and Cys11, respectively, of a second identical monomeric peptide chain.
VIR-683: linked via two cysteine bridges between Cys6 and Cysl1 of one monomeric peptide chain to Cys6 and Cys11, respectively, of a second identical monomeric peptide chain.
VIR-684: linked via two cysteine bridges between Cys6 and Cys11 of one monomeric peptide chain to Cys6 and Cys11, respectively, of a second identical monomeric peptide chain.
VIR-685: linked via two cysteine bridges between Cys6 and Cys11 of one monomeric peptide chain to Cys6 and Cys11, respectively, of a second identical monomeric peptide chain.
VIR-686: linked via an amide bond between Glu11 of one monomeric peptide chain and Ly16 of the second identical monomeric peptide chain.
VIR-687: linked via an amide bond between Glu11 of one unit and Lys16 of the second identical monomeric peptide chain.
VIR-689: linked via an amide bond between Glu11 of one monomeric peptide chain and Lys16 of the second identical monomeric peptide chain.
VIR-690: linked via an amide bond between Glu11 of one monomeric peptide chain and Lys16 of the second identical monomeric peptide chain.
VIR-691: linked via an amide bond between Glu11 of one monomeric peptide chain and Lys16 of the second identical monomeric peptide chain.
VIR-692: linked via an amide bond between Glu11 of one monomeric peptide chain and Lys16 of the second identical monomeric peptide chain.
VIR-693: linked via two cysteine bridges between Cys8 and Cys13 of one monomeric peptide chain to Cys8 and Cys13, respectively, of a second identical monomeric peptide chain.
VIR-694: linked via an amide bond between Glu11 of one monomeric peptide chain and Lys16 of the second identical monomeric peptide chain.
VIR-695: linked via a cysteine bridge between Cys6 of one monomeric peptide chain to Cys6 of the second identical monomeric peptide chain.
VIR-696: linked via a cysteine bridge between Cys6 of one monomeric peptide chain to Cys6 of the second identical monomeric peptide chain.
VIR-697: linked via a cysteine bridge between Cys7 of one monomeric peptide chain to Cys7 of the second identical monomeric peptide chain.
VIR-698: linked via a cysteine bridge between Cys7 of one monomeric peptide chain to Cys7 of the second identical monomeric peptide chain.
VIR-699: linked via a cysteine bridge between Cys6 of one monomeric peptide chain to Cys6 of the second identical monomeric peptide chain.
VIR-700: linked via a cysteine bridge between Cys6 of one monomeric peptide chain to Cys6 of the second identical monomeric peptide chain.
VIR-701: linked via a cysteine bridge between Cys6 of one monomeric peptide chain to Cys6 of the second identical monomeric peptide chain.
VIR-702: linked via a cysteine bridge between Cys6 of one monomeric peptide chain to Cys6 of the second identical monomeric peptide chain.
VIR-703: linked via a cysteine bridge between Cys6 of one monomeric peptide chain to Cys6 of the second identical monomeric peptide chain.
VIR-704: linked via a cysteine bridge between Cys6 of one monomeric peptide chain to Cys6 of the second identical monomeric peptide chain.
VIR-705: linked via a cysteine bridge between Cys6 of one monomeric peptide chain to Cys6 of the second identical monomeric peptide chain.
VIR-706: linked via a cysteine bridge between Cys6 of one monomeric peptide chain to Cys6 of the second identical monomeric peptide chain.
VIR-707: linked via a cysteine bridge between Cys6 of one monomeric peptide chain to Cys6 of the second identical monomeric peptide chain.
VIR-708: linked via a cysteine bridge between Cys6 of one monomeric peptide chain to Cys6 of the second identical monomeric peptide chain.
VIR-709: linked via a cysteine bridge between Cys6 of one monomeric peptide chain to Cys6 of the second identical monomeric peptide chain.
VIR-710: linked via a cysteine bridge between Cys6 of one monomeric peptide chain to Cys6 of the second identical monomeric peptide chain.
VIR-711: linked via a cysteine bridge between Cys6 of one monomeric peptide chain to Cys6 of the second identical monomeric peptide chain.
VIR-712: linked via a cysteine bridge between Cys6 of one monomeric peptide chain to Cys6 of the second identical monomeric peptide chain.
VIR-713: linked via a cysteine bridge between-Cys8 of one monomeric peptide chain to Cys8 of the second identical monomeric peptide chain.
VIR-714: linked via a cysteine bridge between Cys6 of one monomeric peptide chain to Cys6 of the second identical monomeric peptide chain.
VIR-715: linked via a cysteine bridge between Cys6 of one monomeric peptide chain to Cys6 of the second identical monomeric peptide chain.
VIR-716: linked via a cysteine bridge between Cys6 of one monomeric peptide chain to Cys6 of the second identical monomeric peptide chain.
VIR-717: linked via a cysteine bridge between Cys6 of one monomeric peptide chain to Cys6 of the second identical monomeric peptide chain.
VIR-718: linked via a lysine core dendrimer.
VIR-719: linked via a cysteine bridge between Cys6 of one monomeric peptide chain to Cys6 of the second identical monomeric peptide chain.
VIR-720: linked via a cysteine bridge between the carboxyl-terminal Cys residues of the two monomeric peptide chains.
VIR-721: linked via a cysteine bridge between Cys7 of one monomeric peptide chain, which in addition to said cysteine bridge contains an intramolecular cysteine bridge between Cys6 and Cysll, and Cys7 of the second monomeric peptide chain, which also contains an intramolecular cysteine bridge between Cys6 and Cys11.
VIR-722: linked via a cysteine bridge between the carboxy-terminal cysteine residues of the two monomeric peptide chains, which both contain an intramolecular cysteine bridge between their Cys6 and Cys11.
VIR-723: linked via two cysteine bridges between Cys6 and the carboxy-terminal Cys residue of one monomeric peptide chain to Cys6 and the carboxy-terminal Cys residue, respectively, of the second identical monomeric peptide chain. If these oligomeric peptides shall carry further modifications, the monomeric peptide chains are modified as it is described below, prior to the dimerization or, in more general terms, prior to oligomerization. Amidated peptide monomers are obtained by solid phase peptide synthesis using resins carrying an amide linker on which the peptide chain is assembled. Acid cleavage of correspondingly synthesized peptides results in peptide amides. In solution phase synthesis amidated peptides are obtained when the C-terminal amino acid is used as a building block which has a preformed carboxamide at the C-terminus. (Chan W.C. (editors) Fmoc solid phase peptide synthesis: A practical approach, Oxford University Press, Oxford 2000).
Acylated peptide monomers are obtained by the skilled person through converting a peptide with free amino or hydroxy groups using activated acylation reagents derived from carboxylic acids such as acyl halogenides or carboxylic anhydride or other reactive carbonyl compounds to a corresponding acylated peptide. As an alternative, acylation can be achieved using free carboxylic acids which are activated in situ by phosphonium- or uronium-type compounds (Greene T.W., Protective groups in organic chemistry, John Wiley & Sons, New York, 1991; Kocienski P., Protecting groups, Thieme-Verlag, Stuttgart 1994; Seewald N. et al., Peptides: biology and chemistry, Wiley-VCH, Weinheim, 2002).
Alkylated peptide monomers can be obtained by incorporating prealkylated amino acid building blocks when carrying out peptide synthesis on the solid support or in solution. Such amino acids are coupled onto the peptide chains using standard activation protocols known to the skilled person (Chan W.C. (editors) Fmoc solid phase peptide synthesis: A practical approach, Oxford University Press, Oxford 2000). Alkylation may also be achieved after assembly of a peptide chain by using appropriate alkylation methods known to the skilled person (Greene T.W., Protective groups in organic chemistry, John Wiley & Sons, New York, 1991; Kocienski P., Protecting groups, Thieme-Verlag, Stuttgart 1994). Such methods may be applied to reactive groups such as amino-, hydroxyl-, thio- and peptide bonds of the peptide backbone in a partially protected peptide.
Sulfated peptides are obtained by using presulfated building blocks of tyrosine or tyrosine derivatives in solid phase or solution peptide synthesis. O-sulfates remain attached to the hydroxy group during peptide cleavage from the resin when highly acid-labile resins such as 2-chlorotrityl resin are used for synthesis (Seewald N. et al., Peptides: biology and chemistry, Wiley-VCH, Weinheim, 2002).
Pegylated peptides contain pegyl residues bound to functional groups of a peptide. Pegyl residues are characterized as hydrophilic linear or branched polymeric chains with a repeating unit -CH₂-CH₂O-. Pegyl residues are introduced into a peptide after assembly of the peptide chain using suitable functionally modified and reactive pegyl-containing substances. Various activated pegyl groups can be attached by the skilled person to peptides by different activation methods to different side chains or terminal functional groups of a peptide such as amino, carboxyl, hydroxy and thio (Veronese F.M. et al., Bioconjug. Chem., 2001, 12, 62-70; Veronese F.M., Biomaterials, 2001, 22, 405-417).
Phosphorylated peptides can be synthesized by solid phase or solution phase peptide synthesis. Synthesis of phosphorylated peptides is usually achieved by the skilled person utilizing phosphorylated hydroxy amino acid building blocks and/or by post-chain assembly phosphorylation of protected peptides with one or more free hydroxy functional groups (Murray J.S., Biopolymers, 2001, 60, 3-31; Chan W.C. et al. (editors), Fmoc solid phase peptide synthesis: A practical approach, Oxford University Press, Oxford, 2000; Seewald N. et al., Peptides: biology and chemistry, Wiley-VCH, Weinheim, 2002).
Glycosylated peptides can be obtained by the skilled person using glycosylated amino acid building blocks which can be incorporated into solid phase or solution phase synthesis of peptides or by the global post-chain assembly glycosylation approach (Davis B.G., Chem. Rev., 2002, 102, 579-602; Chan W.C. et al. (editors), Fmoc solid phase peptide synthesis: A practical approach, Oxford University Press, Oxford, 2000; Seewald N. et al., Peptides: biology and chemistry, Wiley-VCH, Weinheim, 2002).
The invention also discloses nucleic acid molecules coding for monomeric peptide chains of the oligomeric peptides according to the invention, in particular DNA and RNA, especially cDNA and mRNA.
Subject of the invention are also antibodies specifically binding to peptides of the invention. The term "specifically" is readily understood by the skilled person. In particular, it means that the antibodies do not bind or do essentially not bind related peptides like VIRIP which are not peptides of the invention. A person skilled in the art obtains antibodies against peptides of the invention by routine methods, and will select specific antibodies of the invention by known screening methods.

The invention relates to oligomeric peptides which specifically interact with and bind to the N-terminal region of the envelope protein gp41 of HIV. The term "interact with" and "bind to" is readily understood by the skilled person. By such binding and interaction, oligomeric peptides of the invention block infection of host cells by HIV particles. The present invention also relates to oligomeric peptides which bind to synthetic peptides corresponding to the fusion peptide of gp41 of HZV. A person skilled in the art detects binding and interaction of anyone of the oligomeric peptides of the invention to the synthetic fusion peptlde of gyp41 of HIV by applying quantitative sttucturelactiiviw relationship (QSAR) assays. These assays comprise but are not limited to the detection of the suppression of the hemolytic effect of the synthetic fusion peptide (Mobley P.W. et al., Blochim. (3iophys. Acta, 1992, 1139, 251-256; Gordon L, Biochim. Biophys. Acta, 1992, 1139, 257-274), microcalorimetry (Gohlke H. et al., Angew. Chem. Int. Ed. Engl., 2002, 41, 2644-2676), or NMR-spectrospical techniques which can be chemical shift titration experiments, saturation transfer difference spectroscopy or two dimensional techniques (Meyer et al., Ernst Schering Res. Found. Workshop, 2004, 44, 149-167).
The invention also relates to a medicament containing the oligomeric peptides, nucleic acids or antibodies of the invention. The medicament is preferably provided in galenic formulations for oral, intravenous, intramuscular, intracutaneous, subcutaneous and/or intrathecal administration. In a preferred embodiment, the medicament comprises at least one further therapeutic agent. Said at least one further therapeutic agent can be a viral protease inhibitor, a reverse transcriptase inhibitor, a fusion inhibitor, a cytokine, a cytokine inhibitor, a glycosylation inhibitor or a viral mRNA inhibitor, etc. Preferably, such inhibitors are directed against HIV. Such combined therapeutics are highly relevant in the treatment of AIDS. The peptides, nucleic adds and antibodies of the invention are preferably used in manufacturing of a medicament for the treatment of HIV infections. This comprises all known strains of the retrovirus HN (human immunodeficiency virus), especially the most common strains of HN-1. HIV-1 Is associated with the outbreak of AIDS.
The invention also relates to a diagnostic agent comprising the oligomeric peptides or antibodies of the invention. The diagnostic agent may be used for assay systems for testing isolated plasma, serum, tissue, urine, semen and/or cerebrospinal fluid for HIV infections.
The invention also relates to assay systems which involve at least one of the oligomeric peptides of the invention as a tool to identify substances which bind to the envelope protein gp41 of HIV, in particular the N-terminal fusion peptide of gp41. Such assays can be any system which is suitable to measure the binding of any substance to the fusion peptide either integrated in the entire gp41 protein in isolated, viral, or any other form, or in synthetic form with a length up to 35 amino acid residues starting with the very N-terminus of gp41. In such assays, which can be any spectroscopical, cellular, or radio-ligand assay, the binding of a substance in competition to at least one oligomeric peptide of the invention is measured. As a result of such competition assays using oligomeric peptides of the invention as a tool, the identification of substances with increased affinity and binding site specificity to HIV gp41 is achieved. Such substances have an improved potency to block cellular infection by HIV particles. They can be used as improved therapeutic agents to cure AIDS.

After chemical synthesis of the several oligomeric peptides of the present invention, yields and molecular weights of the products obtained were examined. For all peptides high yields were achieved and the theoretical molecular weights confirmed (see table 1), reflecting the success of the synthesis process. The peptides of the present invention were subjected to various tests.

The first set of experiments concerned the efficacy of the peptides of the present invention to inhibit HIV infection (see table 2). The peptides were tested on the HIV-1 strain NL4-3 and IC₅₀ values were calculated. Peptides with considerable activity where those with an IC₅₀ of equal or below 2000 nM (VIR-673, SEQ ID NO. 4), and those with an IC₅₀ of equal or below 6500 nM (VIR-574, SEQ ID NO.: 2; VIR-577, SEQ ID NO.3) The oligomeric peptides tested had an increased activity in comparison to the native VIRIP (SEQ ID NO. 1); the native VIRIP (SEQ ID NO. 1) was found to have an IC₅₀ of 15,000, if tested with HIV strain NL4-3, respectively. To summarise table 2, the peptides of the present invention displayed a 5-fold to 22-fold increase in anti-HIV activity in comparison to the native VIRIP.

A second set of experiments concerned the stability of the peptides of the present invention in mammalian plasma. Plasma isolated from various animals and humans was spiked with defined concentrations of various peptides of the present invention. The peptides displayed a considerably improved half-life and metabolism in human plasma. The peptides also show an improved stability in plasma of living animals.

In essence, the peptides of the present invention are characterised by their anti-HIV activity, which, expressed as IC₅₀, is equal to or below 6500 nM, whereby the most active peptides have an IC₅₀ of below 2000 nM. Individual peptides of the present invention were found to have IC₅₀ of below 800 nM (see table 1). Peptides of the invention are also characterized by their improved half-life in plasma.

### Example 1: Chemical synthesis of peptides of the present invention

### A. Synthesis of monomeric peptide chains:

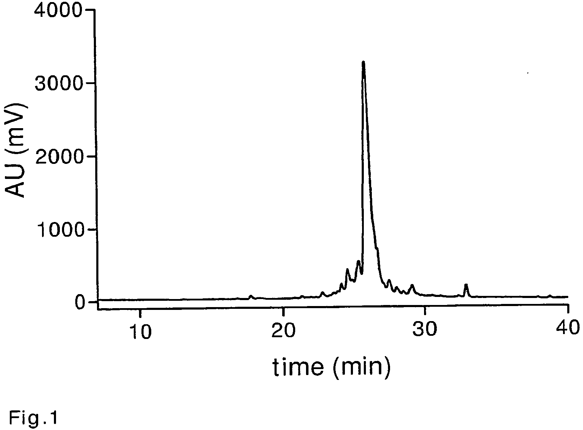

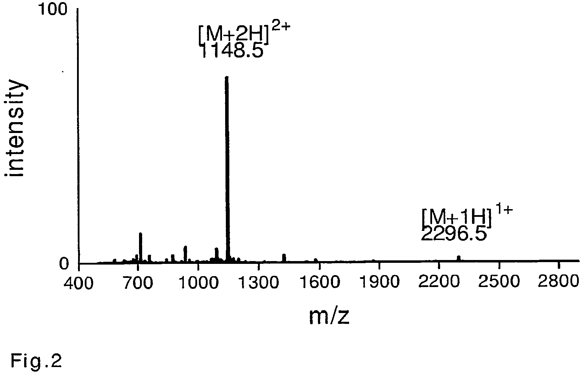

The monomeric peptide chains of the oligomeric peptides according to the invention were chemically synthesized utilizing the principle of solid-phase peptide synthesis and the Fmoc or Boc protective group strategy (Atherton and Sheppard, 1989, Solid Phase Peptide Synthesis, IRL Press; Merrifield, 1986, Solid phase synthesis, Science 232, 341-347), but can also be synthesized with solution phase synthesis or by coupling protected or unprotected fragments of the peptides according to the invention.
As an example, the synthesis of the monomeric peptide chain of the oligomeric peptide VIR-577 [amino acid sequence: LEAIPMCIPPEFLFGKPFVF] (SEQ ID NO. 55) is described here using fluorenylmethoxycarbonyl (Fmoc)-protected amino acids on an automated peptide synthesizer 433A (Applied Biosystems). The synthesis of the monomeric peptide was performed using a preloaded Fmoc-Phe-Wang resin with a loading capacity of 1 mmol/g resin with standard HBTU [(2-(1H-benzotriazol-1-yl)-1,1,3,3-tetramethyluronium-hexafluorophosphate]/HOBt (1-hydroxybenzotriazol) activation with capping cycles using acetic anhydride in N-methylpyrrolidinone (NMP) at a scale of 0.1 mmol. The side chains of the amino acid building blocks used were protected as follows: Glu(OtBu), Lys(Boc), Cys(Trt). Acylation steps for peptide chain assembly were carried out for 15-60 min, and Fmoc groups were deprotected with piperidine in NMP after each acylation. After deprotection of the leucine residue at position 1, the resulting protected peptidyl resin was washed with NMP, 2-propanol and dichloromethane and then dried. The dry resin was treated at room temperature with a fresh mixture of trifluoracetic acid/ethanedithiole/water (94:3:3, vol/vol/vol, 40 ml/g resin) for 2-4.5 h. The mixture was filtrated into ice-cold tert-butylmethylether (TBME) to facilitate precipitation of the peptide. The resulting precipitate was separated by . centrifugation, washed with TBME and dried under vacuum. The crude peptide was analyzed using analytical C18 HPLC and electrospray mass spectrometry (API 100, Perkin Elmer) (Figure 1 + 2)

### B. Oligomerization of monomeric peptide chains to dimeric VIR-577:

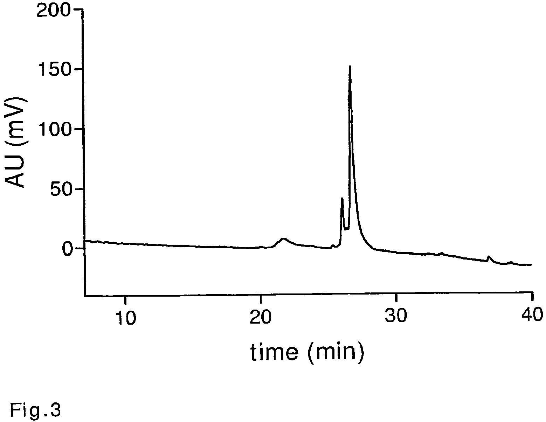

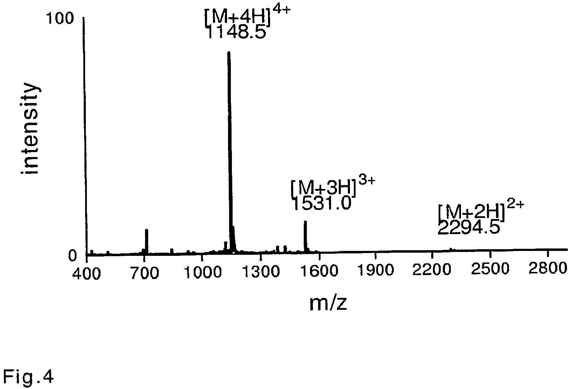

Crude monomeric peptide chains of VIR-577 were dissolved in CH₃CN/H₂O (1:4) at a peptide concentration of 4 mg/ml. 8% DMSO were added to the solution and the pH was adjusted to ∼ 8 by diluted ammonia. The solution was stirred vigorously at room temperature over night. After 18 h the crude mixture was acidified by acetic acid and loaded onto a preparative Vydac C18 column (47x300 mm, 15-20 µm, flow rate 40 ml/min; solvent A, 0.07 volume % TFA; solvent B, 0.07 volume % TFA in acetonitrile/H₂O 80:20 (volume %); UV detection at 215 nm; with the following gradient: 45-70 volume % B in 50 min. The fractions containing the desired pure oligomeric peptide, as detected by mass spectrometry (API 100, Perkin Elmer) and analytical C18 HPLC or, alternatively, capillary zone electrophesis, were pooled and dried by lyophilization. The lyophilized oligomeric peptide was used for analysis of purity and molecular weight by analytical C18 HPLC (Figure 3), capillary zone electrophoresis, and mass spectrometry (Figure 4). The yield of the peptide (LEAIPMCIPPEFLFGKPFVF)₂ (SEQ ID NO. 3) was 7 mg.

### C. Synthesis of lysine-core dendrimers:

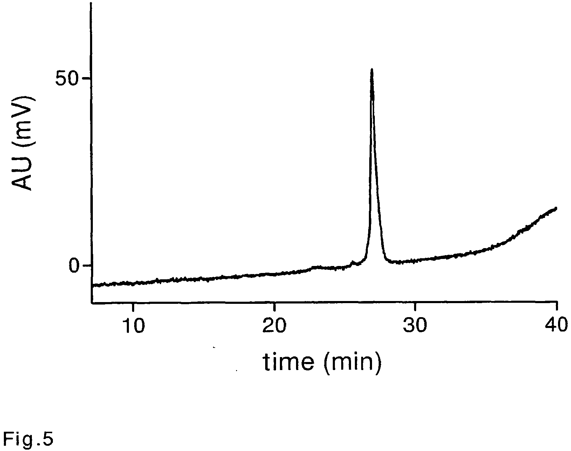

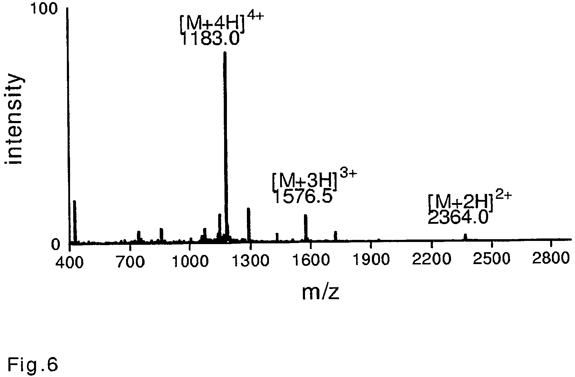

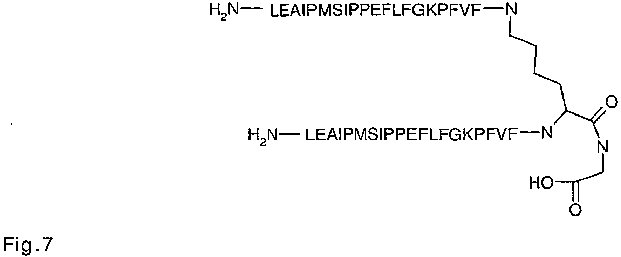

For the synthesis of the lysine-core dendrimers a Fmoc-Gly-Wang resin or a Fmoc-Wang-Ala resin is used. Onto the first amino acid a Fmoc-Lys(Fmoc)-derivative or a Fmoc-Orn(Fmoc) derivative is coupled. After Fmoc-deprotection using 20% piperidine in NMP the resin bears two functional amine groups onto which the synthesis of the linear VIRIP-peptides is carried out by standard Fmoc-cycles. This lysine core can easily be extended by coupling another Fmoc-Lys(Fmoc) or Fmoc-Orn(Fmoc) derivative to the core, generating four free amine groups for the synthesis of the linear peptides. As an example the synthesis of VIR-574 [amino acid sequence: (LEAIPMSIPPEFLFGKPFVF)2-K-G] (SEQ ID NO. 2) is described using fluorenylmethoxycarbonyl (Fmoc)-protected amino acids on an automated peptide synthesizer 433A (Applied Biosystems). The synthesis was performed using a preloaded Fmoc-Gly-Wang resin with a loading capacity of 0.68 mmol/g resin with standard HBTU [(2-(1H-benzotriazol-1-yl)-1,1,3,3-tetramethyluronium-hexafluorophosphate]/HOBt (1-hydroxybenzotriazol) activation with capping cycles using acetic anhydride in N-methylpyrrolidinone (NMP) at a scale of 0.1 mmol. The side chains of the amino acid building blocks used were protected as follows: Glu(OtBu), Lys(Boc), Lys(Fmoc), Ser(tBu). Acylation steps for peptide chain assembly were carried out for 15-60 min, and Fmoc groups were deprotected with piperidine in NMP after each acylation. After deprotection of the leucine residue at position 1, the resulting protected peptidyl resin was washed with NMP, 2-propanol and dichloromethane and then dried. The dry resin was treated at room temperature with a fresh mixture of trifluoracetic acid/ethanedithiole/water (94:3:3, vol/vol/vol, 40 ml/g resin) for 2-4.5 h. The mixture was filtrated into ice-cold tert-butylmethylether (TBME) to facilitate precipitation of the peptide. The resulting precipitate was separated by centrifugation, washed with TBME and dried under vacuum. The crude oligomeric peptide was dissolved in diluted acetic acid and loaded onto a preparative Vydac C18 column (47x300 mm, 15-20 µm, flow rate 40 ml/min; solvent A, 0.07 volume % TFA; solvent B, 0.07 volume % TFA in acetonitrile/H₂O 80:20 (volume %); UV detection at 215 nm; with the following gradient: 45-70 volume % B in 50 min. The fractions containing the desired pure oligomeric peptide, as detected by mass spectrometry (API 100, Perkin Elmer) and analytical C18 HPLC or, alternatively, capillary zone electrophoresis, were pooled and dried by lyophilization. The lyophilized oligomeric peptide was used for analysis of purity and molecular weight by analytical C18 HPLC (Figure 5), capillary zone electrophoresis, and mass spectrometry (Figure 6).
The synthesis process was also adapted to small-scale multiple peptide synthesis. Oligomeric peptides according to the invention having two intermolecular disulfide bonds were treated with air at pH 7.5-8.5, with or without dimethylsulfoxide, and subsequently with iodine to facilitate cysteine bridge formation, starting from precursors with one acetamidomethyl-protected cysteine and one unprotected cysteine to form dimers.

### D. Synthesis of monomeric peptide chains with Acm-protection at the cysteines:

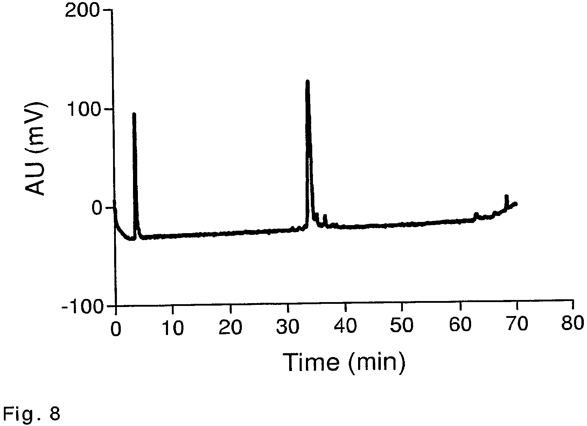

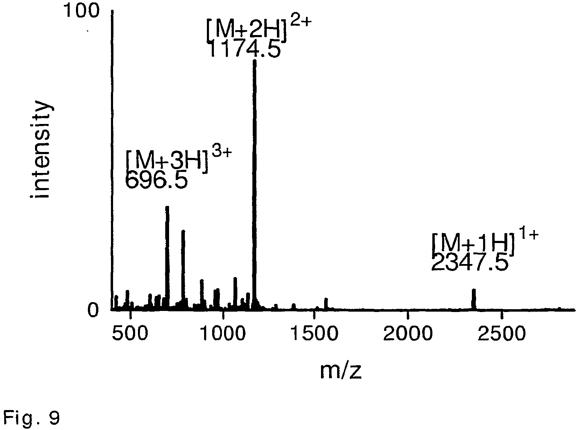

The monomeric peptide chains of the oligomeric peptides according to the invention were chemically synthesized utilizing the principle of solid-phase peptide synthesis and the Fmoc or Boc protective group strategy (Atherton and Sheppard, 1989, Solid Phase Peptide Synthesis, IRL Press; Merrifield, 1986, Solid phase synthesis, Science 232, 341-347), but can also be synthesized with solution phase synthesis or by coupling protected or unprotected fragments of the peptides according to the invention.
As an example, the synthesis of the monomeric Acm-protected peptide chain of the oligomeric peptide VIR-705 [amino acid sequence: LEKIPC(Acm)SIPpEVAFNKPFVF] (SEQ ID NO. 36) is described here using fluorenylmethoxycarbonyl (Fmoc)-protected amino acids on an automated peptide synthesizer 433A (Applied Biosystems). The synthesis of the monomeric peptide was performed using a preloaded Fmoc-Phe-Wang resin with a loading capacity of 1 mmol/g resin with standard HBTU [(2-(1H-benzotriazol-1-yl)-1,1,3,3-tetramethyluronium-hexafluorophosphate]/HOBt (1-hydroxybenzotriazol) activation with capping cycles using acetic anhydride in N-methylpyrrolidinone (NMP) at a scale of 0.1 mmol. The side chains of the amino acid building blocks used were protected as follows: Glu(OtBu), Asn(Trt) Lys(Boc), Ser(tBu), Cys(Acm). Acylation steps for peptide chain assembly were carried out for 15-60 min, and Fmoc groups were deprotected with piperidine in NMP after each acylation. After deprotection of the leucine residue at position 1, the resulting protected peptidyl resin was washed with NMP, 2-propanol and dichloromethane and then dried. The dry resin was treated at room temperature with a fresh mixture of trifluoracetic acid/ethanedithiole/water (94:3:3, vol/vol/vol, 40 ml/g resin) for 2-4.5 h. The mixture was filtrated into ice-cold tert-butylmethylether (TBME) to facilitate precipitation of the peptide. The resulting precipitate was separated by centrifugation, washed with TBME and dried under vacuum. The crude peptide was analyzed using analytical C18 HPLC and electrospray mass spectrometry (API 100, Perkin Elmer) (Figure 8 + 9)

### E. Oligomerization of monomeric Acm-protected peptide chains to dimeric VIR-705:

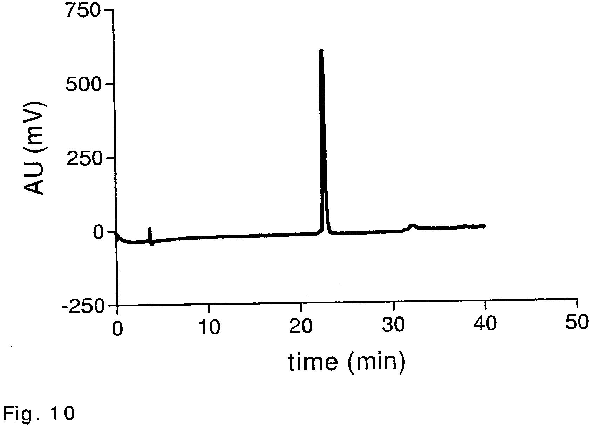

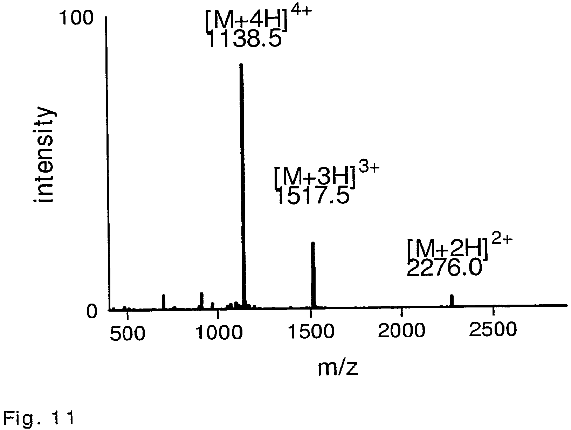

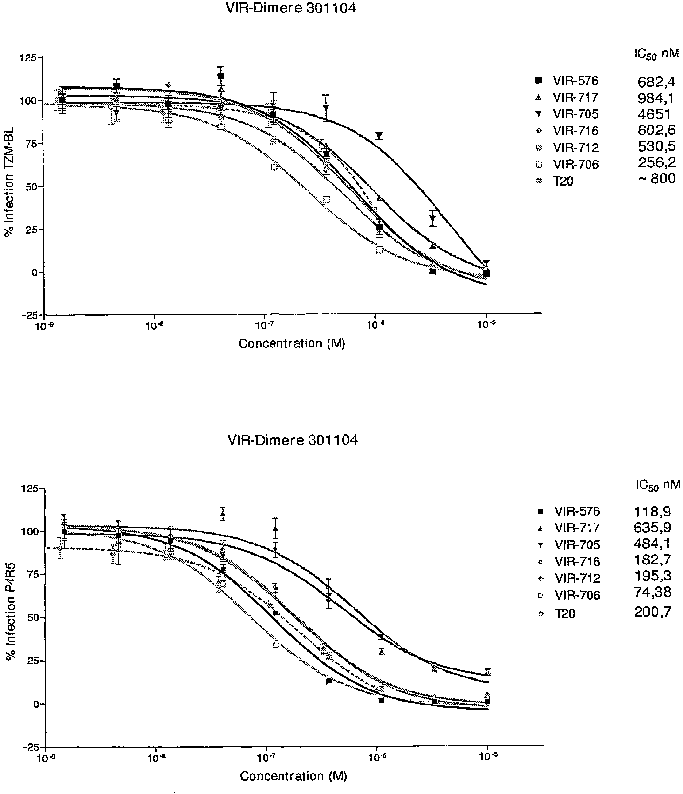

Crude monomeric peptide chains of VIR-577 were dissolved in HOAc/H₂O (4:1) at a peptide concentration of 6 mg/ ml. 4 equivalents of iodine from a 0.05 M I₂ solution in HOAc were added to the solution. The solution was stirred vigorously at room temperature for 60 minutes and the remaining I₂ was reduced with a solution of ascorbic acid. The crude mixture was diluted with 5 volumes of H₂O and loaded onto a preparative Vydac C18 column (47x300 mm, 15-20 µm, flow rate 40 ml/min; solvent A, 0.07 volume % TFA; solvent B, 0.07 volume % TFA in acetonitrile/H₂O 80:20 (volume %); UV detection at 215 nm; with the following gradient: 45-70 volume % B in 50 min. The fractions containing the desired pure oligomeric peptide, as detected by mass spectrometry (API 100, Perkin Elmer) and analytical C18 HPLC or, alternatively, capillary zone electrophesis, were pooled and dried by lyophilization. The lyophilized oligomeric peptide was used for analysis of purity and molecular weight by analytical C18 HPLC (Figure 10), capillary zone electrophoresis, and mass spectrometry (Figure 11). The yield of the peptide (LEKIPCSIPpEVAFNKPFVF)₂ (SEQ ID NO. 36) was 97 mg.
The process for synthesis of the oligomeric peptides according to the invention was adapted to larger scales ranging from 0.5 to 20 mmol yielding purified peptides of the present invention in amounts between 1 g and 5 g.
Using these general synthetic approaches, oligomeric peptides shown in Table 1, among others, were synthesized, purified by chromatographic methods to a degree of up to 98% and analysed:

**Table 1: Yields and molecular weight of synthetic oligomeric peptides.**

| Peptide | Yield [mg] | Molecular weight (calculated) | Molecular weight (determined by mass spectrometry) |
|---|---|---|---|
| VIR-574 | 5 | 4726.4 | 4726.8 |
| VIR-577 | 7 | 4589.6 | 4590.0 |
| VIR-673 | 4 | 5052.0 | 5052.9 |
| VIR-705 | 95 | 4549.5 | 4549.9 |
| VIR-706 | 70 | 4551.4 | 4551.3 |
| VIR-712 | 69 | 4529.5 | 4529.7 |
| VIR-716 | 68 | 4643.7 | 4644.3 |
| VIR-717 | 73 | 4617.5 | 4617.7 |

VIR-577 is a homo-dimer; an intermolecular disulfide bridge occurs at the cysteine at amino acid position 7. VIR-574 is a lysine core linked homo-dimer. VIR-673 is a disulfide bonded homo-dimer with a cysteine residue and a PEG-spacer between the cysteine and the peptide sequence. VIR-705 is a homo-dimer; an intermolecular disulfide bridge occurs at the cysteine at amino acid position 6. VIR-706 is a homo-dimer; an intermolecular disulfide bridge occurs at the cysteine at amino acid position 6. VIR-712 is a homo-dimer; an intermolecular disulfide bridge occurs at the cysteine at amino acid position 6. VIR-716 is a homo-dimer; an intermolecular disulfide bridge occurs at the cysteine at amino acid position 6. VIR-717 is a homo-dimer; an intermolecular disulfide bridge occurs at the cysteine at amino acid position 6.

### Example 2: Inhibition of the HIV infection by the oligoomeric peptides of the present invention

P4-CCR5 indicator cells (Charneau et al., 1994; Journal of Molecular Biology 241, 651-662) expressing the primary CD4 receptor and both major HIV-1 entry cofactors CXCR4 and CCR5, were used to evaluate whether the oligomeric peptides according to the invention are potent inhibitors of HIV-1 infection. These cells contain the ß-galactosidase reporter gene under the control of the HIV-1 promoter. Thus, activation of the ß-galactosidase reporter gene allows to measure the efficiency of HIV-1 infection and thus to quantitate the potency of HIV-1 inhibitors (Detheux M. et al., 2000; Journal of Experimental Medicine 192, 1501-1508; Münch et al., 2002; Antimicrobial Agents and Chemotherapy 46, 982-990).
To perform a typical infection assay, P4-CCR5 cells (Charneau et al., 1994; Journal of Molecular Biology 241, 651-662; Charneau et al., Virology. 1994 205, 247-53) were kept in RPMI 1640 medium supplemented with 10 volume % FCS. This cell line coexpresses CD4 and both HIV-1 coreceptors CCR5 and CXCR4 and contains the ß-galactosidase gene under the control of the HIV-1 promoter. Virus stocks were generated by the calcium coprecipitation method as described (Detheux et al., J Exp Med. 192:1501-8; 2000), and the p24 antigen levels were quantitated with an HIV p24 ELISA kit obtained through the NIH AIDS Reagent Program. Cells were seeded in flat-bottomed 96-well dishes, cultured overnight, and incubated with the different doses of peptide for 2 h before infection with virus containing 1 ng of p24 antigen in a total volume of 50 ml of medium. After overnight incubation, cells were washed twice and cultivated in fresh culture medium without inhibitory peptide. Three days after infection the cells were lysed, and infectivity was quantitated using the Galacto-Light Plus^{Tm} chemiluminescence reporter assay kit (Tropix, Bedford, MA) as recommended by the manufacturer. All infections were performed in quintuplicate.
The results of this assay demonstrate that the oligomeric peptides according to the invention have greatly enhanced anti-HIV-1 activity as compared to VIRIP. Oligomeric peptides of the invention inhibited the infection by the X4-tropic HIV-1 NL4-3 up to 22 fold (Table 2). These data demonstrate that the oligomerization and the specific modifications of the wild-type VIRIP sequence greatly enhance the anti-HIV-1 potency of the oligomeric peptides according to the invention. Below, the IC₅₀ values of peptides of the invention obtained from the described infection assay are provided.

**Table 2: Amino acid sequence and anti-HIV activity.**

| Peptide | Amino acid sequence | SEQ ID NO. | IC50 NL4-3 [nM] |
|---|---|---|---|
| VIR-574 | (LEAIPMSIPPEFLFGKPFVF)₂-K-G | 2 | 3085 |
| VIR-577 | (LEAIPMCIPPEFLFGKPFVF)₂ | 3 | 3270 |
| VIR-673 | (LEAIPMSIPPEFLFGKPFVF-miniPEG-C-amide)₂ | 4 | 664 |
| VIR-705 | (LEKIPCSIPpEVAFNKPFVF)₂ | 36 | 484 |
| VIR-706 | (LEAIPCGIPpEV(D-Tic)FNKPFVF)₂ | 37 | 74 |
| VIR-712 | (N-Me-LEAIPCSIPPEFLFGKPFVF)₂ | 43 | 195 |
| VIR-716 | (N-Me-LEKIPCSIPPEFLFGKPFVF)₂ | 47 | 182 |
| VIR-717 | (N-Me-LEDIPCSIPPEFLFGKPFVF)₂ | 48 | 635 |

VIR-577 is a homo-dimer; an intermolecular disulfide bridge occurs at the cysteine at amino acid position 7. VIR-574 is a lysine core linked homo-dimer. VIR-673 is a disulfide bonded homo-dimer with a cysteine residue and a PEG-spacer between the cysteine and the peptide sequence. VIR-705 is a homo-dimer; an intermolecular disulfide bridge occurs at the cysteine at amino acid position 6. VIR-706 is a homo-dimer; an intermolecular disulfide bridge occurs at the cysteine at amino acid position 6. VIR-712 is a homo-dimer; an intermolecular disulfide bridge occurs at the cysteine at amino acid position 6. VIR-716 is a homo-dimer; an intermolecular disulfide bridge occurs at the cysteine at amino acid position 6. VIR-717 is a homo-dimer; an intermolecular disulfide bridge occurs at the cysteine at amino acid position 6.

### Brief Description of the Drawings:

**Figure 1****:**
   C18 HPLC trace of VIR-577 monomer [LEAIPMCIPPEFLFGKPFVF] (SEQ ID NO. 55). Conditions: Vydac C18 (4.6 x 250 mm, 300 Å, 5 µm, flow rate: 0.8 ml/min, gradient: 10-70 volume % B in 30 min, buffer A: 0.07 volume % TFA, buffer B: 0.05 volume % TFA, 80 volume % acetonitrile).
**Figure 2****:**
   Electrospray-ionization mass spectrum (ESI-MS) of VIR-577 monomer [LEAIPMCIPPEFLFGKPFVF] (SEQ ID NO. 55). The mass spectrum was recorded using a Sciex API 100 mass spectrometer. The molecular ions for [M+2H]²⁺ (m/z 1148.5) and [M+1H]¹⁺ (m/z 2296.5) are indicated.
**Figure 3****:**
   C18 HPLC trace of VIR-577 [ (LEAIPMCIPPEFLFGKPFVF)₂] (SEQ ID NO. 3). Conditions: Vydac C18 (4.6 × 250 mm, 300 Å, 5 µm, flow rate: 0.8 ml/min, gradient: 10-70 volume % B in 30 min, buffer A: 0.07 volume % TFA, buffer B: 0.05 volume % TFA, 80 volume % acetonitrile).
**Figure 4****:**
   Electrospray-ionization mass spectrum (ESI-MS) of VIR-577 [ (LEAIPMCIPPEFLFGKPFVF)₂] (SEQ ID NO. 3). The mass spectrum was recorded using a Sciex API 100 mass spectrometer. The molecular ions for [M+4H] ⁴⁺ (m/z 1148.5), [M+3H]³⁺ (m/z 1531.0) and [M+2H]²⁺ (m/z 2294.5) are indicated.
**Figure 5****:**
   C18 HPLC trace of VIR-574 [ (LEAIPMSIPPEFLFGKPFVF)₂-K-G] (SEQ ID NO. 2). Conditions: Vydac C18 (4.6 × 250 mm, 300 Å, 5 µm, flow rate: 0.8 ml/min, gradient: 10-70 volume % B in 30 min, buffer A: 0.07 volume % TFA, buffer B: 0.05 volume % TFA, 80 volume % acetonitrile).
**Figure 6****:**
   Electrospray-ionization mass spectrum (ESI-MS) of VIR-577 [ (LEAIPMSIPPEFLFGKPFVF)₂-K-G] (SEQ ID NO. 2). The mass spectrum was recorded using a Sciex API 100 mass spectrometer. The molecular ions for [M+4H]⁴⁺ (m/z 1183.0), [M+3H]³⁺ (m/z 1576.5) and [M+2H]²⁺ (m/z 2364.0) are indicated.
**Figure 7****:**
   Chemical Drawing of VIR-574 [(LEAIPMSIPPEFLFGKPFVF)₂-K-G] (SEQ ID NO. 2), as an example for a lysin-core dendrimer.
**Figure 8****:**
   C18 HPLC trace of VIR-705 monomer [LEKIPC(Acm)SIPpEVAFNKPFVF] (SEQ ID NO. 36). Conditions: Vydac C18 (4.6 x 250 mm, 300 Å, 5 µm, flow rate: 0.8 ml/min, gradient: 10-70 volume % B in 60 min, buffer A: 0.07 volume % TFA, buffer B: 0.05 volume % TFA, 80 volume % acetonitrile).
**Figure 9****:**
   Electrospray-ionization mass spectrum (ESI-MS) of VIR-705 monomer [LEKIPC(Acm)SIPpEVAFNKPFVF] (SEQ ID NO. 36). The mass spectrum was recorded using a Sciex API 100 mass spectrometer. The molecular ions for [M+3H]³⁺ (m/z 696.5), [M+2H]²⁺ (m/z 1174.5) and [M+1H]¹⁺ (m/z 2347.5) are indicated.
**Figure 10****:**
   C18 HPLC trace of VIR-705 dimer [(LEKIPCSIPpEVAFNKPFVF)₂] (SEQ ID NO. 36). Conditions: Vydac C18 (4.6 × 250 mm, 300 Å, 5 µm, flow rate: 0.8 ml/min, gradient: 10-70 volume % B in 30 min, buffer A: 0.07 volume % TFA, buffer B: 0.05 volume % TFA, 80 volume % acetonitrile).
**Figure 11****:**
   Electrospray-ionization mass spectrum (ESI-MS) of VIR-705 dimer [(LEKIPCSIPpEVAFNKPFVF)₂] (SEQ ID NO. 36). The mass spectrum was recorded using a Sciex API 100 mass spectrometer. The molecular ions for [M+4H]⁴⁺ (m/z 1138.5), [M+3H]³⁺ (m/z 1517.5) and [M+2H]²⁺ (m/z 2276.0) are indicated.

### Abbreviations:

- AIDS:: aquired immuno-deficiency syndrome
- Boc:: tert-butyloxycarbonyl
- CXCR4:: CXC chemokine receptor 4
- CCR5:: CC chemokine recptor 5
- ESI-MS:: electrospray ionization-mass spectrometry
- FP:: fusion peptide
- HIV:: human immunodeficiency virus
- HPLC:: high performance liquid chromatography
- HR-1, HR-2:: heptad repeat 1, 2
- MALDI-TOF:: matrix-assisted laser desorption/ionization-time-of-flight
- Mini-PEG:: -NH-(CH₂)₂-O-(CH₂)₂-O-CH₂-CO-NH-(CH₂)₂-O-(CH₂)₂-O-CH₂-CO-NH₂
- NMR:: nuclear magnetic resonance
- Oic:: octahydroindolyl-2-carboxylic acid
- PEG:: pegyl, polyoxyethyleneglycol
- QSAR:: quantitative structure-activity relationship
- tBu:: tert-butyl
- TFA:: trifluoroacetc acid
- Tic:: 1,2,3,4-tetrahydroisoquinoline-3-carboxylic acid
- Trt:: trityl

## Claims

1. Oligomeric peptides, with biological activity against infection by HIV, wherein the peptides are selected from the group consisting of the peptides consisting of the amino acid sequences
| | | |
|---|---|---|
| VIR-574 | (LEAIPMSIPPEFLFGKPFVF)₂-K-G | SEQ ID NO. 2 |
| | a lysine core linked homo-dimer | |
| VIR-577 | (LEAIPMCIPPEFLFGKPFVF)₂ | SEQ ID NO. 3 |
| | a homo-dimer with an intermolecular disulfide bridge at the cysteine at amino acid position 7 | |
| VIR-673 | (LEAIPMSIPPEFLFGKPFVF-miniPEG-C-amide)₂ | SEQ ID NO. 4 |
| | a disulfide bonded homo-dimer with a cysteine residue and a PEG-spacer between the cysteine and the peptide sequence | |
| VIR-674 | (LEAIPCSIPPCVA(D-Tic)NKP(D-TiC)FVF) | SEQ ID NO. 5 |
| | linked via two cysteine bridges between Cys6 and Cys11 of one monomeric peptide chain to Cys6 and Cys11, respectively, of a second identical monomeric peptide chain | |
| VIR-677 | (LEAIPMCIPPECFFNKPFVF)₂ | SEQ ID NO. 8 |
| | linked via two cysteine bridges between Cys7 and Cys12 of one monomeric peptide chain to Cys7 and Cys12, respectively, of a second identical monomeric peptide chain | |
| VIR-678 | (LEAIPMCIPPECLFGKPFVF)₂ | SEQ ID NO. 9 |
| | linked via two cysteine bridges between Cys7 and Cys12 of one monomeric peptide chain to Cys7 and Cys12, respectively, of a second identical monomeric peptide chain | |
| VIR-679 | (LEAIPCSIPPCVFFGKPFVF)₂ | SEQ ID NO. 10 |
| | linked via two cysteine bridges between Cys6 and Cys11 of one monomeric peptide chain to Cys6 and Cys11, respectively, of a second identical monomeric peptide chain | |
| VIR-680 | (LEAIPCSIPPCFLFGKPFVF)₂ | SEQ ID NO. 11 |
| | linked via two cysteine bridges between Cys6 and Cys11 of one monomeric peptide chain to Cys6 and Cys11, respectively, of a second identical monomeric peptide chain | |
| VIR-681 | (LEAIPCSIPpCVGFGKPFVF)₂ | SEQ ID NO. 12 |
| | linked via two cysteine bridges between Cys6 and Cys11 of one monomeric peptide chain to Cys6 and Cys11, respectively, of a second identical monomeric peptide chain | |
| VIR-682 | (LEAIPCSIPpCVFFNKPFVF)₂ | SEQ ID NO. 13 |
| | linked via two cysteine bridges between Cys6 and Cys11 of one monomeric peptide chain to Cys6 and Cys11, respectively, of a second identical monomeric peptide chain | |
| VIR-683 | (LEAIPCSIPpCFLFNKPFVF)₂ | SEQ ID NO. 14 |
| | linked via two cysteine bridges between Cys6 and Cys11 of one monomeric peptide chain to Cys6 and Cys11, respectively, of a second identical monomeric peptide chain | |
| VIR-684 | (LEDIPCSIPpCVAFNKPFVF)₂ | SEQ ID NO. 15 |
| | linked via two cysteine bridges between Cys6 and Cys11 of one monomeric peptide chain to Cys6 and Cys11, respectively, of a second identical monomeric peptide chain | |
| VIR-685 | (LEKIPCSIPpCVAFNKPFVF)₂ | SEQ ID NO. 16 |
| | linked via two cysteine bridges between Cys6 and Cys11 of one monomeric peptide chain to Cys6 and Cys11, respectively, of a second identical monomeric peptide chain | |
| VIR-686 | (LEAIPMGxPpEV(D-Tic)FNKPFVF)₂ | SEQ ID NO. 17 |
| | linked via an amide bond between Glu11 of one monomeric peptide chain and Lys16 of the second identical monomeric peptide chain | |
| VIR-687 | (LEAIPMGIPpEV(L-Tic)FNKPFVF)₂ | SEQ ID NO. 18 |
| | linked via an amide bond between Glu11 of one unit and Lys16 of the second identical monomeric peptide chain | |
| VIR-688 | (LEKIPMSIPpEV(D-Tic)FNKPFVF)₂ | SEQ ID NO. 19 |
| VIR-689 | (LEKTPMSIPpEV(L-Tic)FNKPFVF)₂ | SEQ ID NO. 20 |
| | linked via an amide bond between Glu11 of one monomeric peptide chain and Lys16 of the second identical monomeric peptide chain | |
| | | |
| VIR-690 | (LEKIPIGIPpEV(D-Tic)FNKPFVF)₂ | SEQ ID NO. 21 |
| | linked via an amide bond between Glu11 of one monomeric | |
| | peptide chain and Lys16 of the second identical monomeric peptide chain | |
| VIR-691 | (LEKIPIGIPpEV(L-Tic)FNKPFVF)₂ | SEQ ID NO. 22 |
| | linked via an amide bond between Glu11 of one monomeric peptide chain and Lys16 of the second identical monomeric peptide chain | |
| VIR-693 | (LEAIPMSCPPEFCFGKPFVF)₂ | SEQ ID NO. 24 |
| | linked via two cysteine bridges between Cys8 and Cys13 of one monomeric peptide chain to Cys8 and Cys13, respectively, of a second identical monomeric peptide chain | |
| VIR-695 | (LEAIPCSIPPEVA(D-Tic)NKP(D-Tic)FVF)₂ | SEQ ID NO. 26 |
| | linked via a cysteine bridge between Cys6 of one monomeric peptide chain to Cys6 of the second identical monomeric peptide chain | |
| VIR-696 | (LEAIPCSIPpE(3,3-diphenylalanine)AFNKPFVF)₂ | SEQ ID NO. 27 |
| | linked via a cysteine bridge between Cys6 of one monomeric peptide chain to Cys6 of the second identical monomeric peptide chain | |
| VIR-697 | (LEAIPMCIPPEVFFNKPFVF)₂ | SEQ ID NO. 28 |
| | linked via a cysteine bridge between Cys7 of one monomeric peptide chain to Cys7 of the second identical monomeric peptide chain | |
| VIR-698 | (LEAIPMCIPPEVLFGKPFVF)₂ | SEQ ID NO. 29 |
| | linked via a cysteine bridge between Cys7 of one monomeric peptide chain to Cys7 of the second identical monomeric peptide chain | |
| VIR-699 | (LEAIPCSIPPEVFFGKPFVF)₂ | SEQ ID NO. 30 |
| | linked via a cysteine bridge between Cys6 of one monomeric peptide chain to Cys6 of the second identical monomeric peptide chain | |
| VIR-700 | (LEAIPCSIPPEFLFGKPFVF)₂ | SEQ ID NO. 31 |
| | linked via a cysteine bridge between Cys6 of one monomeric peptide chain to Cys6 of the second identical monomeric peptide chain | |
| VIR-701 | (LEAIPCSIPpEVGFGKPFVF)₂ | SEQ ID NO. 32 |
| | linked via a cysteine bridge between Cys6 of one monomeric peptide chain to Cys6 of the second identical monomeric peptide chain | |
| VIR-702 | (LEAIPCSIPpEVFFNKPFVF)₂ | SEQ ID NO. 33 |
| | linked via a cysteine bridge between Cys6 of one monomeric peptide chain to Cys6 of the second identical monomeric peptide chain | |
| VIR-703 | (LEAIPCSIPpEFLFNKPFVF)₂ | SEQ ID NO. 34 |
| | linked via a cysteine bridge between Cys6 of one monomeric peptide chain to Cys6 of the second identical monomeric peptide chain | |
| VIR-704 | (LEDIPCSIPpEVAFNKPFVF)₂ | SEQ ID NO. 35 |
| | linked via a cysteine bridge between Cys6 of one monomeric peptide chain to Cys6 of the second identical monomeric peptide chain | |
| VIR-705 | (LEKIPCSIPpEVAFNKPFVF)₂ | SEQ ID NO. 36 |
| | linked via a cysteine bridge between Cys6 of one monomeric peptide chain to Cys6 of the second identical monomeric peptide chain | |
| VIR-706 | (LEAIPCGIPpEV(D-Tic)FNKPFVF)₂ | SEQ ID NO. 37 |
| | linked via a cysteine bridge between Cys6 of one monomeric peptide chain to Cys6 of the second identical monomeric peptide chain | |
| VIR-707 | (LEAIPCGIPpEV(L-Tic)FNKPFVF)₂ | SEQ ID NO. 38 |
| | linked via a cysteine bridge between Cys6 of one monomeric peptide chain to Cys6 of the second identical monomeric peptide chain | |
| VIR-708 | (LEKIPCSIPpEV(D-Tic)FNKPFVF)₂ | SEQ ID NO. 39 |
| | linked via a cysteine bridge between Cys6 of one monomeric peptide chain to Cys6 of the second identical monomeric peptide chain | |
| VIR-709 | (LEKIPCSIPpEV(L-Tic)FNKPFVF)₂ | SEQ ID NO. 40 |
| | linked via a cysteine bridge between Cys6 of one monomeric | |
| | peptide chain to Cys6 of the second identical monomeric peptide chain | |
| VIR-710 | (LEKIPCGIPpEV(D-Tic)FNKPFVF)₂ | SEQ ID NO. 41 |
| | linked via a cysteine bridge between Cys6 of one monomeric peptide chain to Cys6 of the second identical monomeric peptide chain | |
| VIR-711 | (LEKIPCGIPpEV(L-Tic)FNKPFVF)₂ | SEQ ID NO. 42 |
| | linked via a cysteine bridge between Cys6 of one monomeric peptide chain to Cys6 of the second identical monomeric peptide chain | |
| VIR-712 | (N-Me-LEAIPCSIPPEFLFGKPFVF)₂ | SEQ ID NO. 43 |
| | linked via a cysteine bridge between Cys6 of one monomeric peptide chain to Cys6 of the second identical monomeric peptide chain | |
| VIR-713 | (LEAIPMSCPPEFLFGKPFVF)₂ | SEQ ID NO. 44 |
| | linked via a cysteine bridge between Cys8 of one monomeric peptide chain to Cys8 of the second identical monomeric peptide chain | |
| VIR-714 | (LEAIPCSIPPEFLFGKPFVF-(miniPEG)2-amide)₂ | SEQ ID NO. 45 |
| | linked via a cysteine bridge between Cys6 of one monomeric peptide chain to Cys6 of the second identical monomeric peptide chain | |
| VIR-715 | (N-Me-LEAIPCSIPPEFLFGKPFVF-(miniPEG)2-amide)₂ | SEQ ID NO. 46 |
| | linked via a cysteine bridge between Cys6 of one monomeric peptide chain to Cys6 of the second identical monomeric peptide chain | |
| VIR-716 | (N-Me-LEKIPCSIPPEFLFGKPFVF)₂ | SEQ ID NO. 47 |
| | linked via a cysteine bridge between Cys6 of one monomeric peptide chain to Cys6 of the second identical monomeric peptide chain | |
| VIR-717 | (N-Me-LEDIPCSIPPEFLFGKPFVF)₂ | SEQ ID NO. 48 |
| | linked via a cysteine bridge between Cys6 of one monomeric peptide chain to Cys6 of the second identical monomeric peptide chain | |
| VIR-718 | (LEKIPIGIPpEV(L-Tic)FNKPFVF)₂-KA | SEQ ID NO. 49 |
| | linked via a lysine core dendrimer | |
| VIR-719 | (N-Me-LEAIPCSIPpEFLFGKPFVF)₂ | SEQ ID NO. 50 |
| | linked via a cysteine bridge between Cys6 of one monomeric peptide chain to Cys6 of the second identical monomeric peptide chain. | |
| | | |
| VIR-720 | (LEKIPIGIPpEV(L-Tic)FNKPFVF-miniPEG-C)₂ | SEQ ID NO. 51 |
| | linked via a cysteine bridge between the carboxyl-terminal Cys residues of the two monomeric peptide chains | |
| VIR-723 | (LEAIPCSIPPEFLFGKPFVF-(miniPEG)2-C)₂ | SEQ ID NO. 54 |
| | linked via two cysteine bridges between Cys6 and the carboxy-terminal Cys residue of one monomeric peptide chain to Cys6 and the carboxy-terminal Cys residue, respectively, of the second identical monomeric | |

2. Oligomeric peptide according to claim 1, wherein the leucine residue at amino acid position 1 and the glutamic acid at amino acid position 2 are covalently linked by an N-alkylated amide bond or by an ester bond or by a reduced peptide bond or by a retro-inverso peptide bond or by an N-alkylated retro-inverso peptide bond.

3. Antibodies binding specifically to the oligomeric peptides according to anyone of the claim 1 or 2.

4. A medicament comprising at least one of the oligomeric peptides according to claim 1 to 2 and/or antibodies of claim 3.

5. The medicament of claim 4 in galenic formulations for oral, intravenous, intramuscular, intracutaneous, subcutaneous and/or intrathecal administration.

6. The medicament of claim 4 or 5 comprising at least one further therapeutic agent.

7. The medicament of claim 6, wherein the said at least one further therapeutic agent is a viral protease inhibitor, a reverse transcriptase inhibitor, a fusion inhibitor, a cytokine, a cytokine inhibitor, a glycosylation inhibitor or a viral mRNA inhibitor.

8. Use of the oligomeric peptides according to claim 1 or 2 for the manufacturing of a medicament for the treatment of HIV infections.

9. An assay for determining molecules capable of interaction with the fusion peptide of HIV, comprising at least one oligomeric peptide according claim 1 or 2.

10. Use of an oligomeric peptide according claim 1 or 2 in an assay according to claim 9.

11. A diagnostic agent comprising at least one oligomeric peptide according to claim 1 or 2 or antibodies according to claim 3.

12. Use of the diagnostic agent according to claim 11 for testing isolated blood, plasma, tissue, urine, semen and/or cerebrospinal fluid for HIV infection.

## Patentansprüche

1. Oligomere Peptide mit biologischer Aktivität gegen Infektion durch HIV, wobei die Peptide aus der Gruppe ausgewählt sind, die aus den Peptiden besteht, welche aus den folgenden Aminosäuren bestehen:
| | |
|---|---|
| VIR-574 (LEAIPMSIPPEFLFGKPFVF)₂-K-G | SEQ ID Nr. 2 |
| ein über einen Lysinkern verknüpftes Homodimer | |
| VIR-577 (LEAIPMCIPPEFLFGKPFVF)₂ | SEQ ID Nr. 3 |
| ein Homodimer mit einer intermolekularen Disulfidbrücke am Cystein auf der Aminosäureposition 7 | |
| VIR-673 (LEAIPMSIPPEFLFGKPFVF-miniPEG-C-amid)₂ | SEQ ID Nr. 4 |
| ein disulfidgebundenes Homodimer mit einem Cysteinrest und einem PEG-Spacer zwischen dem Cystein und der Peptidsequenz | |
| VIR-674 (LEAIPCSIPPCVA(D-Tic)NKP(D-Tic)FVF) | SEQ ID Nr. 5 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-677 (LEAIPMCIPPECFFNKPFVF)₂ | SEQ ID Nr. 8 |
| verknüpft über zwei Cysteinbrücken zwischen Cys7 und Cys12 einer monomeren Peptidkette nach Cys7 und Cys12 einer zweiten, identischen monomeren Peptidkette | |
| VIR-678 (LEAIPMCIPPECLFGKPFVF)₂ | SEQ ID Nr. 9 |
| verknüpft über zwei Cysteinbrücken zwischen Cys7 und Cys12 einer monomeren Peptidkette nach Cys7 und Cys12 einer zweiten, identischen monomeren Peptidkette | |
| VIR-679 (LEAIPCSIPPCVFFGKPFVF)₂ | SEQ ID Nr. 10 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-680 (LEAIPCSIPPCFLFGKPFVF)₂ | SEQ ID Nr. 11 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-681 (LEAIPCSIPpCVGFGKPFVF)₂ | SEQ ID Nr. 12 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-682 (LEAIPCSIPpCVFFNKPFVF)₂ | SEQ ID Nr. 13 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-683 (LEAIPCSIPpCFLFNKPFVF)₂ | SEQ ID Nr. 14 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-684 (LEDIPCSIPpCVAFNKPFVF)₂ | SEQ ID Nr. 15 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-685 (LEKIPCSIPpCVAFNKPFVF)₂ | SEQ ID Nr. 16 |
| verknüpft über zwei Cysteinbrücken zwischen Cys6 und Cys11 einer monomeren Peptidkette nach Cys6 und Cys11 einer zweiten, identischen monomeren Peptidkette | |
| VIR-686 (LEAIPMGIPpEV(D-Tic)FNKPFVF)₂ | SEQ ID Nr. 17 |
| verknüpft über eine Amidbindung zwischen Glu11 einer monomeren Peptidkette und Lys16 der zweiten, identischen monomeren Peptidkette | |
| VIR-687 (LEAIPMGIPpEV(L-Tic)FNKPFVF)₂ | SEQ ID Nr. 18 |
| verknüpft über eine Amidbindung zwischen Glu11 einer Einheit und Lys16 der zweiten, identischen monomeren Peptidkette | |
| VIR-688 (LEKIPMSIPpEV(D-Tic)FNKPFVF)₂ | SEQ ID Nr. 19 |
| VIR-689 (LEKIPMSIPpEV(L-Tic)FNKPFVF)₂ | SEQ ID Nr. 20 |
| verknüpft über eine Amidbindung zwischen Glu11 einer monomeren Peptidkette und Lys16 der zweiten, identischen monomeren Peptidkette | |
| VIR-690 (LEKIPIGIPpEV(D-Tic)FNKPFVF)₂ | SEQ ID Nr. 21 |
| verknüpft über eine Amidbindung zwischen Glu11 einer monomeren Peptidkette und Lys16 der zweiten, identischen monomeren Peptidkette | |
| VIR-691 (LEKIPIGIPpEV(L-Tic)FNKPFVF)₂ | SEQ ID Nr. 22 |
| verknüpft über eine Amidbindung zwischen Glu11 einer monomeren Peptidkette und Lys16 der zweiten, identischen monomeren Peptidkette | |
| VIR-693 (LEAIPMSCPPEFCFGKPFVF)₂ | SEQ ID Nr. 24 |
| verknüpft über zwei Cysteinbrücken zwischen Cys8 und Cys13 einer monomeren Peptidkette nach Cys8 und Cys13 einer zweiten, identischen monomeren Peptidkette | |
| VIR-695 (LEAIPCSIPPEVA(D-Tic)NKP(D-Tic)FVF)₂ | SEQ ID Nr. 26 |
| verknüpft über eine Cysteinbrücke zwischen Cys6 einer monomeren Peptidkette nach Cys6 der zweiten, identischen monomeren Peptidkette | |
| VIR-696 (LEAIPCSIPpE(3,3-Diphenylalanin)AFNKPFVF)₂ | SEQ ID Nr. 27 |
| verknüpft über eine Cysteinbrücke zwischen Cys6 einer monomeren Peptidkette nach Cys6 der zweiten, identischen monomeren Peptidkette | |
| VIR-697 (LEAIPMCIPPEVFFNKPFVF)₂ | SEQ ID Nr. 28 |
| verknüpft über eine Cysteinbrücke zwischen Cys7 einer monomeren Peptidkette nach Cys7 der zweiten, identischen monomeren Peptidkette | |
| VIR-698 (LEAIPMCIPPEVLFGKPFVF)₂ | SEQ ID Nr. 29 |
| verknüpft über eine Cysteinbrücke zwischen Cys7 einer monomeren Peptidkette nach Cys7 der zweiten, identischen monomeren Peptidkette | |
| VIR-699 (LEAIPCSIPPEVFFGKPFVF)₂ | SEQ ID Nr. 30 |
| verknüpft über eine Cysteinbrücke zwischen Cys6 einer monomeren Peptidkette nach Cys6 der zweiten, identischen monomeren Peptidkette | |
| VIR-700 (LEAIPCSIPPEFLFGKPFVF)₂ | SEQ ID Nr. 31 |
| verknüpft über eine Cysteinbrücke zwischen Cys6 einer monomeren Peptidkette nach Cys6 der zweiten, identischen monomeren Peptidkette | |
| VIR-701 (LEAIPCSIPpEVGFGKPFVF)₂ | SEQ ID Nr. 32 |
| verknüpft über eine Cysteinbrücke zwischen Cys6 einer monomeren Peptidkette nach Cys6 der zweiten, identischen monomeren Peptidkette | |
| VIR-702 (LEAIPCSIPpEVFFNKPFVF)₂ | SEQ ID Nr. 33 |
| verknüpft über eine Cysteinbrücke zwischen Cys6 einer monomeren Peptidkette nach Cys6 der zweiten, identischen monomeren Peptidkette | |
| VIR-703 (LEAIPCSIPpEFLFNKPFVF)₂ | SEQ ID Nr. 34 |
| verknüpft über eine Cysteinbrücke zwischen Cys6 einer monomeren Peptidkette nach Cys6 der zweiten, identischen monomeren Peptidkette | |
| VIR-704 (LEDIPCSIPpEVAFNKPFVF)₂ | SEQ ID Nr. 35 |
| verknüpft über eine Cysteinbrücke zwischen Cys6 einer monomeren Peptidkette nach Cys6 der zweiten, identischen monomeren Peptidkette | |
| VIR-705 (LEKIPCSIPpEVAFNKPFVF)₂ | SEQ ID Nr. 36 |
| verknüpft über eine Cysteinbrücke zwischen Cys6 einer monomeren Peptidkette nach Cys6 der zweiten, identischen monomeren Peptidkette | |
| VIR-706 (LEAIPCGIPpEV(D-Tic)FNKPFVF)₂ | SEQ ID Nr. 37 |
| verknüpft über eine Cysteinbrücke zwischen Cys6 einer monomeren Peptidkette nach Cys6 der zweiten, identischen monomeren Peptidkette | |
| VIR-707 (LEAIPCGIPpEV(L-Tic)FNKPFVF)₂ | SEQ ID Nr. 38 |
| verknüpft über eine Cysteinbrücke zwischen Cys6 einer monomeren Peptidkette nach Cys6 der zweiten, identischen monomeren Peptidkette | |
| VIR-708 (LEKIPCSIPpEV(D-Tic)FNKPFVF)₂ | SEQ ID Nr. 39 |
| verknüpft über eine Cysteinbrücke zwischen Cys6 einer monomeren Peptidkette nach Cys6 der zweiten, identischen monomeren Peptidkette | |
| VIR-709 (LEKIPCSIPpEV(L-Tic)FNKPFVF)₂ | SEQ ID Nr. 40 |
| verknüpft über eine Cysteinbrücke zwischen Cys6 einer monomeren Peptidkette nach Cys6 der zweiten, identischen monomeren Peptidkette | |
| VIR-710 (LEKIPCGIPpEV(D-Tic)FNKPFVF)₂ | SEQ ID Nr. 41 |
| verknüpft über eine Cysteinbrücke zwischen Cys6 einer monomeren Peptidkette nach Cys6 der zweiten, identischen monomeren Peptidkette | |
| VIR-711 (LEKIPCGIPpEV(L-Tic)FNKPFVF)₂ | SEQ ID Nr. 42 |
| verknüpft über eine Cysteinbrücke zwischen Cys6 einer monomeren Peptidkette nach Cys6 der zweiten, identischen monomeren Peptidkette | |
| VIR-712 (N-Me-LEAIPCSIPPEFLFGKPFVF)₂ | SEQ ID Nr. 43 |
| verknüpft über eine Cysteinbrücke zwischen Cys6 einer monomeren Peptidkette nach Cys6 der zweiten, identischen monomeren Peptidkette | |
| VIR-713 (LEAIPMSCPPEFLFGKPFVF)₂ | SEQ ID Nr. 44 |
| verknüpft über eine Cysteinbrücke zwischen Cys8 einer monomeren Peptidkette nach Cys8 der zweiten, identischen monomeren Peptidkette | |
| VIR-714 (LEAIPCSIPPEFLFGKPFVF-(miniPEG)₂-amid)₂ | SEQ ID Nr. 45 |
| verknüpft über eine Cysteinbrücke zwischen Cys6 einer monomeren Peptidkette nach Cys6 der zweiten, identischen monomeren Peptidkette | |
| VIR-715 (N-Me-LEAIPCSIPPEFLFGKPFVF-(miniPEG)₂-amid)₂ | SEQ ID Nr. 46 |
| verknüpft über eine Cysteinbrücke zwischen Cys6 einer monomeren Peptidkette nach Cys6 der zweiten, identischen monomeren Peptidkette | |
| VIR-716 (N-Me-LEKIPCSIPPEFLFGKPFVF)₂ | SEQ ID Nr. 47 |
| verknüpft über eine Cysteinbrücke zwischen Cys6 einer monomeren Peptidkette nach Cys6 der zweiten, identischen monomeren Peptidkette | |
| VIR-717 (N-Me-LEDIPCSIPPEFLFGKPFVF)₂ | SEQ ID Nr. 48 |
| verknüpft über eine Cysteinbrücke zwischen Cys6 einer monomeren Peptidkette nach Cys6 der zweiten, identischen monomeren Peptidkette | |
| VIR-718 (LEKIPIGIPpEV(L-Tic)FNKPFVF)₂-KA | SEQ ID Nr. 49 |
| verknüpft über ein Lysinkern-Dendrimer | |
| VIR-719 (N-Me-LEAIPCSIPpEFLFGKPFVF)₂ | SEQ ID Nr. 50 |
| verknüpft über eine Cysteinbrücke zwischen Cys6 einer monomeren Peptidkette nach Cys6 der zweiten, identischen monomeren Peptidkette | |
| VIR-720 (LEKIPIGIPpEV(L-Tic)FNKPFVF-(miniPEG-C)₂ | SEQ ID Nr. 51 |
| verknüpft über eine Cysteinbrücke zwischen den carboxyterminalen Cys-Resten der beiden monomeren Peptidketten | |
| VIR-723 (LEAIPCSIPPEFLFGKPFVF-(miniPEG)₂-C)₂ | SEQ ID Nr. 54 |
| verknüpft über zwei Cysteinbrücken zwischen Cys6 und dem carboxyterminalen Cys-Rest einer monomeren Peptidkette nach Cys6 und dem carboxyterminalen Cys-Rest der zweiten, identischen monomeren Peptidkette. | |

2. Oligomeres Peptid gemäß Anspruch 1, wobei der Leucinrest auf Aminosäureposition 1 und der Glutaminsäurerest auf Aminosäureposition 2 durch eine N-alkylierte Amidbindung oder durch eine Esterbindung oder durch eine reduzierte Peptidbindung oder durch eine retro-inverso-Peptidbindung oder durch eine N-alkylierte retro-inverso-Peptidbindung kovalent miteinander verknüpft sind.

3. Antikörper, die spezifisch an die oligomeren Peptide gemäß einem der Ansprüche 1 oder 2 binden.

4. Medikament, das wenigstens eines der oligomeren Peptide gemäß Anspruch 1 oder 2 und/oder Antikörper gemäß Anspruch 3 umfasst.

5. Medikament gemäß Anspruch 4 in galenischen Zubereitungen für die orale, intravenöse, intramuskuläre, intrakutane, subkutane und/oder intrathekale Verabreichung.

6. Medikament gemäß Anspruch 4 oder 5, das wenigstens ein weiteres therapeutisches Mittel umfasst.

7. Medikament gemäß Anspruch 6, wobei das wenigstens eine weitere therapeutische Mittel ein Virus-Protease-Inhibitor, ein Reverse-Transcriptase-Inhibitor, ein Fusionsinhibitor, ein Cytokin, ein Cytokin-Inhibitor, ein Glycosylierungsinhibitor oder ein Virus-mRNA-Inhibitor ist.

8. Verwendung der oligomeren Peptide gemäß Anspruch 1 oder 2 zur Herstellung eines Medikaments zur Behandlung von HIV-Infektionen.

9. Assay zur Bestimmung von Molekülen, die zu einer Wechselwirkung mit dem Fusionspeptid von HIV befähigt sind, umfassend wenigstens ein oligomeres Peptid gemäß Anspruch 1 oder 2.

10. Verwendung eines oligomeren Peptids gemäß Anspruch 1 oder 2 in einem Assay gemäß Anspruch 9.

11. Diagnostisches Mittel, das wenigstens ein oligomeres Peptid gemäß Anspruch 1 oder 2 oder Antikörper gemäß Anspruch 3 umfasst.

12. Verwendung des diagnostischen Mittels gemäß Anspruch 11 zum Testen von isoliertem Blut, Plasma, Gewebe, Urin, Sperma und/oder Liquor auf HIV-Infektion.

## Revendications

1. Peptides oligomériques, ayant une activité biologique contre une infection par le VIH, dans lesquels les peptides sont choisis dans le groupe constitué par les peptides consistant en les séquences d'acides aminés
| | | |
|---|---|---|
| VIR-574 | (LEAIPMSIPPEFLFGKPFVF)₂-K-G | SEQ ID N° 2 |
| | un homo-dimère lié à un noyau lysine | |
| VIR-577 | (LEAIPMCIPPEFLFGKPFVF)₂ | SEQ ID N° 3 |
| | un homo-dimère avec un pont disulfure intermoléculaire au niveau de la cystéine en position 7 d'acide aminé | |
| VIR-673 | (LEAIPMSIPPEFLFGKPFVF-miniPEG-C-amide)₂ | SEQ ID N° 4 |
| | un homo-dimère lié par un disulfure avec un résidu cystéine et un espaceur PEG entre la cystéine et la séquence peptidique | |
| VIR-674 | (LEAIPCSIPPCVA(D-Tic)NKP(D-Tic)FVF) | SEQ ID N° 5 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-677 | (LEAIPMCIPPECFFNKPFVF)₂ | SEQ ID N° 8 |
| | lié via deux ponts cystéine entre Cys7 et Cys12 d'une chaîne peptidique monomérique à Cys7 et Cys12, respectivement, d'une seconde chaîne peptidique monomérique identique | |
| VIR-678 | (LEAIPMCIPPECLFGKPFVF)₂ | SEQ ID N° 9 |
| | lié via deux ponts cystéine entre Cys7 et Cys12 d'une chaîne peptidique monomérique à Cys7 et Cys12, respectivement, d'une seconde chaîne peptidique monomérique identique | |
| VIR-679 | (LEAIPCSIPPCVFFGKPFVF)₂ | SEQ ID N° 10 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-680 | (LEAIPCSIPPCFLFGKPFVF)₂ | SEQ ID N° 11 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-681 | (LEAIPCSIPpCVGFGKPFVF)₂ | SEQ ID N° 12 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-682 | (LEAIPCSIPpCVFFNKPFVF)₂ | SEQ ID N° 13 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-683 | (LEAIPCSIPpCFLFNKPFVF)₂ | SEQ ID N° 14 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-684 | (LEDIPCSIPpCVAFNKPFVF)₂ | SEQ ID N° 15 |
| | Lié via deux ponts cystéine entre Cys6 et Cys11 d'une chaîne peptidique monomérique à Cys6 et Cys11, respectivement, d'une seconde chaîne peptidique monomérique identique | |
| VIR-685 | (LEKIPCSIPpCVAFNKPFVF)₂ | SEQ ID N° 16 |
| | lié via deux ponts cystéine entre Cys6 et Cys11 d'une chaîne peptidique monomérique à Cys6 et Cys11, respectivement, d'une seconde chaîne peptidique monomérique identique | |
| VIR-686 | (LEAIPMGIPpEV(D-Tic)FNKPFVF)₂ | SEQ ID N° 17 |
| | lié via une liaison amide entre Glu11 d'une chaîne peptidique monomérique et Lys16 de la seconde chaîne | |
| VIR-687 | (LEAIPMGIPpEV(L-Tic)FNKPFVF)₂ | SEQ ID N° 18 |
| | lié via une liaison amide entre Glu11 d'un motif et Lys16 de la seconde chaîne peptidique monomérique identique | |
| VIR-688 | (LEKIPMSIPpEV(D-Tic)FNKPFVF)₂ | SEQ ID N° 19 |
| VIR-689 | (LEKIPMSIPpEV(L-Tic)FNKPFVF)₂ | SEQ ID N° 20 |
| | lié via une liaison amide entre Glu11 d'une chaîne peptidique monomérique et Lys16 de la seconde chaîne peptidique monomérique identique | |
| VIR-690 | (LEKIPIGIPpEV(D-Tic)FNKPFVF)₂ | SEQ ID N° 21 |
| | lié via une liaison amide entre Glu11 d'une chaîne peptidique monomérique et Lys16 de la seconde chaîne peptidique monomérique identique | |
| VIR-691 | (LEKIPIGIPpEV(L-Tic)FNKPFVF)2 | SEQ ID N° 22 |
| | lié via une liaison amide entre Glu11 d'une chaîne peptidique monomérique et Lys16 de la seconde chaîne peptidique monomérique identique | |
| VIR-693 | (LEAIPMSCPPEFCFGKPFVF)₂ | SEQ ID N° 24 |
| | lié via deux ponts cystéine entre Cys8 et Cys13 d'une chaîne peptidique monomérique à Cys8 et Cys13, respectivement, d'une seconde chaîne peptidique monomérique identique | |
| VIR-695 | (LEAIPCSIPPEVA (D-Tic) NKP (D-Tic) FVF)₂ | SEQ ID N° 26 |
| | lié via un pont cystéine entre Cys6 d'une chaîne peptidique monomérique à Cys6 de la seconde chaîne peptidique monomérique identique | |
| VIR-696 | (LEAIPCSIPpE (3,3-diphénylalanine) AFNKPFVF)₂ | SEQ ID N° 27 |
| | lié via un pont cystéine entre Cys6 d'une chaîne peptidique monomérique à Cys6 de la seconde chaîne peptidique monomérique identique | |
| VIR-697 | (LEAIPMCIPPEVFFNKPFVF)₂ | SEQ ID N° 28 |
| | lié via un pont cystéine entre Cys7 d'une chaîne peptidique monomérique à Cys7 de la seconde chaîne peptidique monomérique identique | |
| VIR-698 | (LEAIPMCIPPEVLFGKPFVF)₂ | SEQ ID N° 29 |
| | lié via un pont cystéine entre Cys7 d'une chaîne peptidique monomérique à Cys7 de la seconde chaîne peptidique monomérique identique | |
| VIR-699 | (LEAIPCSIPPEVFFGKPFVF)2 | SEQ ID N° 30 |
| | lié via un pont cystéine entre Cys6 d'une chaîne peptidique monomérique à Cys6 de la seconde chaîne peptidique monomérique identique | |
| VIR-700 | (LEAIPCSIPPEFLFGKPFVF)₂ | SEQ ID N° 31 |
| | lié via un pont cystéine entre Cys6 d'une chaîne peptidique monomérique à Cys6 de la seconde chaîne peptidique monomérique identique | |
| VIR-701 | (LEAIPCSIPpEVGFGKPFVF)₂ | SEQ ID N° 32 |
| | lié via un pont cystéine entre Cys6 d'une chaîne peptidique monomérique à Cys6 de la seconde chaîne peptidique monomérique identique | |
| VIR-702 | (LEAIPCSIPpEVFFNKPFVF)₂ | SEQ ID N° 33 |
| | lié via un pont cystéine entre Cys6 d'une chaîne peptidique monomérique à Cys6 de la seconde chaîne peptidique monomérique identique | |
| VIR-703 | (LEAIPCSIPpEFLFNKPFVF)₂ | SEQ ID N° 34 |
| | lié via un pont cystéine entre Cys6 d'une chaîne peptidique monomérique à Cys6 de la seconde chaîne peptidique monomérique identique | |
| VIR-704 | (LEDIPCSIPpEVAFNKPFVF)₂ | SEQ ID N° 35 |
| | lié via un pont cystéine entre Cys6 d'une chaîne peptidique monomérique à Cys6 de la seconde chaîne peptidique monomérique identique | |
| VIR-705 | (LEKIPCSIPpEVAFNKPFVF) 2 | SEQ ID N° 36 |
| | lié via un pont cystéine entre Cys6 d'une chaîne peptidique monomérique à Cys6 de la seconde chaîne peptidique monomérique identique | |
| VIR-706 | (LEAIPCGIPpEV(D-Tic)FNKPFVF)₂ | SEQ ID N° 37 |
| | lié via un pont cystéine entre Cys6 d'une chaîne peptidique monomérique à Cys6 de la seconde chaîne peptidique monomérique identique | |
| VIR-707 | (LEAIPCGIPpEV (L-Tic) FNKPFVF)₂ | SEQ ID N° 38 |
| | lié via un pont cystéine entre Cys6 d'une chaîne peptidique monomérique à Cys6 de la seconde chaîne peptidique monomérique identique | |
| VIR-708 | (LEKIPCSIPpEV (D-Tic) FNKPFVF)₂ | SEQ ID N° 39 |
| | lié via un pont cystéine entre Cys6 d'une chaîne peptidique monomérique à Cys6 de la seconde chaîne peptidique monomérique identique | |
| VIR-709 | (LEKIPCSIPpEV (L-Tic) FNKPFVF)₂ | SEQ ID N° 40 |
| | lié via un pont cystéine entre Cys6 d'une chaîne peptidique monomérique à Cys6 de la seconde chaîne peptidique monomérique identique | |
| VIR-710 | (LEKIPCGIPpEV (D-Tic) FNKPFvF)₂ | SEQ ID N° 41 |
| | lié via un pont cystéine entre Cys6 d'une chaîne peptidique monomérique à Cys6 de la seconde chaîne peptidique monomérique identique | |
| VIR-711 | (LEKIPCGIPpEV (L-Tic) FNKPFVF)₂ | SEQ ID N° 42 |
| | lié via un pont cystéine entre Cys6 d'une chaîne peptidique monomérique à Cys6 de la seconde chaîne peptidique monomérique identique | |
| VIR-712 | (N-Me-LEAIPCSIPPEFLFGKPFVF)₂ | SEQ ID N° 43 |
| | lié via un pont cystéine entre Cys6 d'une chaîne peptidique monomérique à Cys6 de la seconde chaîne peptidique monomérique identique | |
| VIR-713 | (LEAIPMSCPPEFLFGKPFVF)₂ | SEQ ID N° 44 |
| | lié via un pont cystéine entre Cys8 d'une chaîne peptidique monomérique à Cys8 de la seconde chaîne peptidique monomérique identique | |
| VIR-714 | (LEAIPCSIPPEFLFGKPFVF- (miniPEG) 2-amide)₂ | SEQ ID N° 45 |
| | lié via un pont cystéine entre Cys6 d'une chaîne peptidique monomérique à Cys6 de la seconde chaîne peptidique monomérique identique | |
| VIR-715 | (N-Me-LEAIPCSIPPEFLFGKPFVF- (miniPEG) 2-amide)₂ | SEQ ID N° 46 |
| | lié via un pont cystéine entre Cys6 d'une chaîne peptidique monomérique à Cys6 de la seconde chaîne peptidique monomérique identique | |
| VIR-716 | (N-Me-LEKIPCSIPPEFLFGKPFVF)₂ | SEQ ID N° 47 |
| | lié via un pont cystéine entre Cys6 d'une chaîne peptidique monomérique à Cys6 de la seconde chaîne peptidique monomérique identique | |
| VIR-717 | (N-Me-LEDIPCSIPPEFLFGKPFVF)₂ | SEQ ID N° 48 |
| | lié via un pont cystéine entre Cys6 d'une chaîne peptidique monomérique à Cys6 de la seconde chaîne peptidique monomérique identique | |
| VIR-718 | (LEKIPIGIPpEV (L-Tic) FNKPFVF)₂-KA | SEQ ID N° 49 |
| | lié via un dendrimère à noyau de lysine | |
| VIR-719 | (N-Me-LEAIPCSIPpEFLFGKPFVF)₂ | SEQ ID N° 50 |
| | lié via un pont cystéine entre Cys6 d'une chaîne peptidique monomérique à Cys6 de la seconde chaîne peptidique monomérique identique | |
| VIR-720 | (LEKIPIGIPpEV (L-Tic) FNKPFVF-miniPEG-C)₂ | SEQ ID N° 51 |
| | lié via un pont cystéine entre les résidus Cys carboxy-terminaux des deux chaînes peptidiques monomériques | |
| VIR-723 | (LEAIPCSIPPEFLFGKPFVF- (miniPEG) 2-C)₂ | SEQ ID N° 54 |
| | lié via deux ponts cystéine entre Cys6 et le résidu Cys carboxy-terminal d'une chaîne peptidique monomérique à Cys6 et le résidu Cys carboxy-terminal, respectivement, de la seconde chaîne peptidique monomérique identique | |

2. Peptide oligomérique selon la revendication 1, dans lequel le résidu leucine à la position d'acide aminé 1 et l'acide glutamique à la position d'acide aminé 2 sont liés de manière covalente par une liaison amide N-alkylé ou par une liaison ester ou par une liaison peptidique réduite ou par une liaison peptidique rétro-inverso ou par une liaison peptidique rétro-inverso N-alkylé.

3. Anticorps se liant spécifiquement aux peptides oligomériques selon l'une quelconque de la revendication 1 ou 2.

4. Médicament comprenant au moins l'un des peptides oligomériques selon la revendication 1 à 2 et/ou des anticorps de la revendication 3.

5. Médicament selon la revendication 4, dans des formulations galéniques pour une administration orale, intraveineuse, intramusculaire, intra-cutanée, sous-cutanée et/ou intrathécale.

6. Médicament selon la revendication 4 ou 5, comprenant au moins un agent thérapeutique supplémentaire.

7. Médicament selon la revendication 6, dans lequel ledit au moins un agent thérapeutique supplémentaire est un inhibiteur de protéase virale, un inhibiteur de transcriptase inverse, un inhibiteur de fusion, une cytokine, un inhibiteur de cytokine, un inhibiteur de glycosylation ou un inhibiteur d'ARNm viral.

8. Utilisation des peptides oligomériques selon la revendication 1 ou 2 pour la fabrication d'un médicament destiné au traitement d'infections par VIH.

9. Dosage permettant de déterminer des molécules capables d'interaction avec le peptide de fusion du VIH comprenant au moins un peptide oligomérique selon la revendication 1 ou 2.

10. Utilisation d'un peptide oligomérique selon la revendication 1 ou 2, dans un dosage selon la revendication 9.

11. Agent diagnostique comprenant au moins un peptide oligomérique selon la revendication 1 ou 2, ou des anticorps selon la revendication 3.

12. Utilisation de l'agent diagnostique selon la revendication 11, pour une analyse de sang isolé, plasma, tissu, urine, sperme et/ou liquide céphalorachidien pour dépister une infection par le VIH.
